# EUROPEAN PATENT SPECIFICATION

(11) **EP 3 897 455 B1**
(45) Date of publication and mention of the grant of the patent: **26.04.2023**
(21) Application number: 19827709.7
(22) Date of filing: 19.12.2019
(51) Int. Cl.: A61F 2/08

(54) **MEDICAL FASTENING DEVICE FOR THE FASTENING OF GRAFTS**
MEDIZINISCHE BEFESTIGUNGSVORRICHTUNG ZUR BEFESTIGUNG VON TRANSPLANTATEN
DISPOSITIF DE FIXATION MÉDICAL POUR LA FIXATION DE GREFFES

(30) Priority: 19.12.2018 EP 18382946
(43) Date of publication of application: 27.10.2021
(73) Proprietor: Abanza Tecnomed, S.L., 31192 Mutilva, Navarra (ES)
(72) Inventor: ABASCAL RUBIO, José Manuel, 31192 Mutilva, Navarra (ES); ABASCAL AZANZA, Juan, 31192 Mutilva, Navarra (ES)
(74) Representative: ABG Intellectual Property Law, S.L.
(86) International application number: PCT/EP2019/086213
(87) International publication number: WO 2020/127667

(56) References cited:
- US-A1- 2006 189 991
- US-A1- 2008 051 795
- US-A1- 2013 006 278
- US-A1- 2013 304 099
- US-A1- 2014 277 185
- US-A1- 2017 143 470
- US-A1- 2017 172 755

## Description

### Object of the Invention

The present invention relates to the field of traumatology. In particular, the present invention relates to a medical fastening device for the fastening of at least one graft in at least one bone tunnel. This invention is intended for the reconstruction of connective tissue, such as tendons and ligaments, of the knee joint or other parts of the body.

### Background of the Invention

In the field of traumatology, one of the most common injuries consists of tears in the ligaments of joints. A ligament is a band of longitudinal connective tissue configured for joining bones to one another within a joint.

One of the most common tears in sports medicine is tearing of the anterior cruciate ligament (ACL) of the knee. A person whose ACL has sustained a complete tear presents both an anteroposterior instability and a rotational instability that prevents playing sports again.

A preferred option today for repairing a torn or injured ACL is the use of fibrous connective tissue obtained from the actual patient, mainly autografts of the semitendinosus and gracilis tendons.

In any event, to repair a damaged ACL, the graft is inserted in tibial and femoral bone tunnels. In particular, a currently widespread practice consists of suspending the bent end of the graft in the femoral bone tunnel or femoral bone tunnels by means of cortical buttons and using interference screws, with or without a plug/sleeve, for fastening the free ends in the tibial bone tunnel.

This practice requires having a sufficient volume of graft, so autografts of both the semitendinosus and gracilis tendons are normally used.

The semitendinosus-gracilis tendons have a mean length of around 260 mm, so when two grafts are provided and folded once, a graft length and thickness which are sufficient for the repair are obtained.

There are different studies which demonstrate that there are fewer sequelae for the patient when only the semitendinosus is used, without removing the gracilis tendon. The following are examples of said studies:
Ardern CL, Webster KE, Taylor NF, Feller JA. (2010) "Hamstring strength recovery after hamstring tendon harvest for anterior cruciate ligament reconstruction: a comparison between graft types*",* and Segawa H, Omori G, Koga Y, Kameo T, Iida S, Tanaka M. (2002) "Rotational muscle strength of the limb after anterior cruciate ligament reconstruction using semitendinosus and gracilis tendon*".*

For this reason, a second practice has become common in this type of repair, said practice consisting of joining the free ends of a single semitendinosus graft, which is folded up in order to configure a graft of three or four branches joined by sutures. This provides a sufficient volume and allows using cortical buttons for suspension of the semitendinosus autograft both in the femoral bone tunnel and in the tibial bone tunnel.

However, there are studies demonstrating that the use of knots, sutures, and cortical buttons can cause the repair to fail due to graft tension loss, both by sliding in the knots and sutures and by the movement of the cortical button, which may even make its way into the bone tunnel. The following are examples of said studies:
Barrow AE, Pilia M, Guda T et al. (2014) "Femoral suspension devices for anterior cruciate ligament reconstruction: do adjustable loops lengthen?*"* and Johnson JS, Smith SD, Laprade CM et al. (2014) "A biomechanical comparison of femoral cortical suspension devices for soft tissue anterior cruciate ligament reconstruction under high loads*".*

The interference screw is a fastening device which directly restrains the graft against the internal wall of the bone tunnel located in the porous trabecular bone. Therefore, the screw is located longitudinally contiguous to the graft, restraining it against the wall of the bone tunnel. A screwing guide which goes through the interference screw and a screwdriver are used for this fastening.

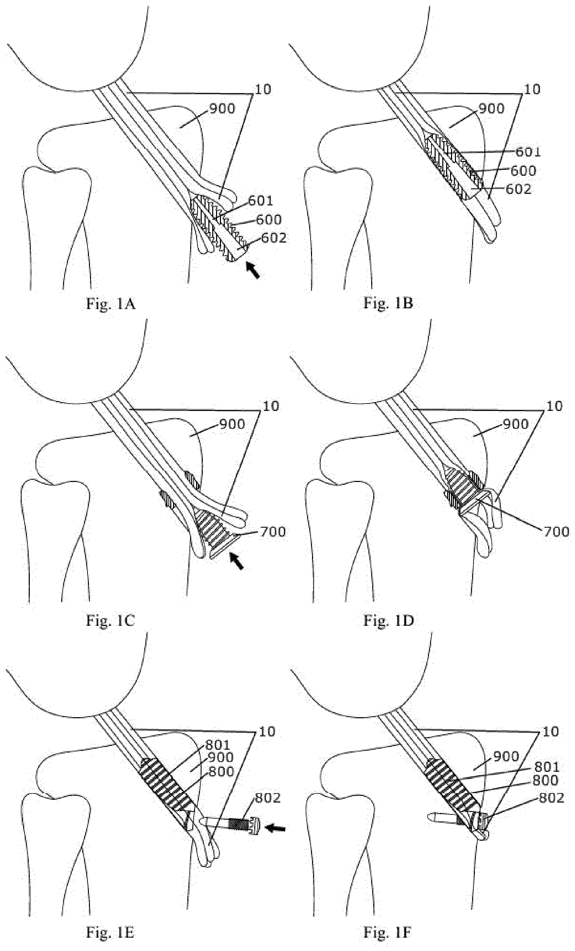

Figure 1A shows an interference screw (600) which, like most interference screws, comprises two contiguous longitudinal through conduits (601, 602) with a smaller diameter. In particular, the distal conduit (601), which has the smallest diameter of the two, is intended for introducing the screwing guide. The proximal conduit (602), which has the largest diameter of the two, is configured with internal polyhedral faces for engaging the screwdriver, with no other use for such conduits being contemplated. Figure 1A shows, in the section of the screw, both conduits (601, 602). Interference screws (600) are fastening devices designed for restraining the graft against the internal wall of the bone tunnel. The internal conduits are secondary elements for guiding and screwing in the screw in the bone tunnel.

A risk associated with the use of an interference screw is that the trabecular bone has a porosity of up to 90%. Therefore, due to the inconsistency of the porous bone of the tibia, these interference screws may be subject to migrations and not correctly retain the graft inside the tunnel, with the subsequent failure of the repair.

Another complication associated with interference screws is that they are screwed directly onto the graft, risking damage thereto, and that they do not allow accurately controlling the tension with which the graft is fastened. Furthermore, this device may cause the widening of the bone tunnel, which slows down and hinders correct osseointegration of the graft.

In this sense, in the current state of the art, there is a need to improve the performance of current fastening devices in relation to ultimate tensile strength, graft tension loss, the possibility of migration of the device into the bone tunnel, efficacy of the fastening of a single semitendinosus graft without the need to extract the gracilis tendon, and resistance to sliding of the graft, in the case of interference screws.

To solve the described problems associated with the use of interference screws and cortical buttons, various patents disclosing fastening devices for ACL repair have been developed. However, the complications associated with these devices continue to condition the mechanical properties of the joint after reconstruction, with the interference screw and cortical button still being the most used devices. Several of said patents are identified below.

For the purpose of mitigating the risk of damaging the graft, US 6517579 B1 describes a technical solution consisting of the fastening of grafts by means of a screw which is introduced in a plug/sleeve with external longitudinal channels in which the grafts are positioned, such that when the screw is introduced in the longitudinal central conduit existing in the plug, the grafts are restrained between the external face of the plug and the wall of the bone tunnel.

This type of technical solution prevents the graft from coming into contact with the screw while screwing it in, which does in fact occur with interference screws without a plug/sleeve. However, this technical solution suffers the primary problem associated with interference screws: when the grafts are restrained between the external face of the plug and the actual wall of the bone tunnel, due to the inconsistency of the porous bone of the walls of the tibial bone tunnel, the device does not provide sufficient retaining force, and the graft may slide into the tibial bone tunnel.

Patent document US20160100936 describes a screw that is screwed coaxial to a ferrule housing the graft. Figures 1C and 1D illustrate the device of said patent application. As can be seen, the screw is screwed directly onto the graft which, as in the case of the interference screw, may damage it.

Patent document WO 2006/091278 A1 describes a screw which is introduced in the bone tunnel and restrains the graft against the internal wall of the tunnel, while at the same time a second screw, with a washer, which enters perpendicular to the cortical bone and is screwed into the angled face of the first screw, restrains the graft a second time.

In this configuration, the reinforcing screw must separate and necessarily pass between the fascicles of the graft to perform fastening, therefore:
- the area being formed on the actual angled face of the screw decreases due to the space occupied by the actual reinforcing screw, and accordingly, the fastening surface is very small, and
- the use of this device involves fitting the graft between the washer and a limited and open tightening area, resulting in a possible incomplete fastening and/or possible damage to the graft.

Patent document US 2006052787 A1 proposes a solution similar to the one proposed in WO 2006/091278 A1, but the screw is replaced with a non-threaded restraining element. Figures 1E and 1F illustrate the device of said patent application. As can be seen, the graft (10) necessarily passes between the external face of the restraining element (801) and the wall of the bone tunnel of the bone, to then bend towards the angled face of the restraining element, with the screw (802), with the washer, pressing the graft (10) against said element.

Patent document WO 2015/169978 A1 describes a fastening for grafts with a longitudinal conduit and a screwing element, and a fastening passage exclusively in the proximal upper rim of the ferrule. Figures 2A to 2E illustrate the device of said patent application. In practice, this device has displayed the following limitations:
- Although the proximal upper rim provides the graft with a pronounced retaining bend, it provides a limited restraining passage which leaves part of the graft unrestrained, reducing the resistance to sliding of the restrained graft, and for this reason the device has to be resized so as to restrain the entire graft.
- The use of direct mechanical restraining devices positioned on the cortical bone, of the type described in international patent application WO 2015/169978 A1, does not allow ACL repair with a single semitendinosus graft, since in order to achieve sufficient thickness, it is folded with a 3-strand or 4-strand configuration, which is too short for a cortical fastening.

The patent application document US-A1 2013/304099 describes a fixation device for securing a transplant in a bone tunnel.

The patent application document US-A1 2008/051795 describes a tissue fixation device preferably used to secure a ligament or graft within a prepared bone tunnel.

Therefore, there is a need to provide a solution which allows fastening at least one graft inside the bone tunnel, improving the performance of current devices, providing the necessary rigidity and force, and solving the described problems.

### Description of the Invention

The present invention proposes a solution to the preceding problems by means of a medical fastening device for fastening a graft according to claim 1, and a system for inserting a medical fastening device according to claim 14. Preferred embodiments of the invention are defined in the dependent claims.

In a first inventive aspect, there is provided *a medical fastening device for the fastening of a graft, wherein the medical fastening device is suitable for being inserted in a bone tunnel of a bone, and wherein said medical fastening device comprises*
- *a first fastening element,*
- *a second fastening element, comprising:*
   ∘ *a first through conduit with a first end and a second end, wherein said first through conduit is extended along a first longitudinal axis, said first through conduit is configured for receiving the graft at its first end and for housing the graft inside same and at its second end,*
   ∘ *a second fastening conduit with a first end and a second end, wherein said second fastening conduit is extended along a second longitudinal axis,*

   *wherein the first through conduit and the second fastening conduit are arranged inside the second fastening element such that the first longitudinal axis and the second longitudinal axis define an angle, α, with one another other than 0*
   *wherein the second end of the first through conduit and the second end of the second fastening conduit are joined defining an annular proximal surface,*
*such that when the first fastening element is housed and fastened in the fastening conduit, an annular fastening passage is defined between the first fastening element and the annular proximal surface.*

In one embodiment, the first longitudinal axis coincides with the axial axis of symmetry of the first through conduit. In another embodiment compatible with any of the preceding embodiments, the second fastening conduit is configured for housing and fastening the first fastening element. In another embodiment compatible with any of the preceding embodiments, the second fastening conduit is extended along the second longitudinal axis.

Throughout this document, graft will be understood as:
- any type of autograft from the actual patient,
- any type of allograft or xenograft from a donor, or
- any type of manufactured graft, flexible material, or synthetic implant.

Throughout this document, the first end of each element of the medical fastening device will be understood to mean the distal end of said element, i.e., the end of the element farthest away from the surgeon at the time of being inserted in the bone tunnel. On the other hand, the second end of each element of the medical fastening device will be understood to mean the proximal end of said element, i.e., the end of the element closest to the surgeon at the time of being inserted in the bone tunnel. On the other hand, it will also be understood that both the first through conduit and the second fastening conduit are hollow.

In that sense, a proximal element or the proximal end is, respectively, an element or the area located in the closest gripping position of the second fastening element for the use thereof by a surgeon. Therefore, when the second fastening element is introduced in a bone tunnel, the proximal end is the area closest to the surgeon and farthest away from the bone tunnel. A distal element or the distal area is, respectively, an element or the area located in the position that is the farthest away for use thereof by any surgeon, and therefore the closest to the bone tunnel. Accordingly, when the second fastening element is introduced in a bone tunnel, the distal area is the area farthest away from the surgeon and the closest to the bone tunnel.

The first through conduit of the second fastening element is configured for receiving the graft through one of the ends of the through conduit and leading said graft therethrough until at least one of the ends of the graft is located in the inner part or face of the annular proximal surface.

The annular proximal surface is located at the second end of the medical fastening device. In particular, the annular proximal surface is a rim which joins the second end of the first through conduit and the second end of the second fastening conduit. Advantageously, the annular proximal surface guides the graft, such that the latter parts from the first longitudinal axis with a retaining bend. As a result of said rim, the retaining bend can be formed in any part of the perimeter of the rim, reinforcing the fastening.

The inner face of the annular proximal surface is part of the annular fastening passage and advantageously allows the fastening of several grafts with a fastening passage that is larger than the one in the mentioned elements of the state of the art. Therefore, the device of the present invention is configured for fastening one or more grafts.

It must be understood that the annular fastening passage is annular because the arrangement of the annular proximal surface continuously prolongs the perimeter defining the junction of the second end of the first through conduit and the second end of the second fastening conduit.

The inner face of the surface of the annular fastening passage is the fastening surface. The fastening surface is the surface fastening the graft, such that the graft is housed in the annular fastening passage and settled on the surfaces making up the fastening surface.

Therefore, the annular fastening passage is the space or volume that is defined between the first fastening element and the inner face of the annular proximal surface of the second end of the second fastening element, when the first fastening element is housed in the second fastening conduit, such that when the graft is housed in the annular fastening passage, and in turn settled between the surfaces making up the fastening passage, it allows fastening and immobilizing the graft. The path of the graft can be linear, curved, zigzag, bent, or be of any other shape indicated in the medical literature. Advantageously, said annular fastening passage assures that fastening is complete and performed in a simpler and more reliable manner compared with the devices of the state of the art.

In one embodiment, *the annular proximal surface* is prolonged along the entire annular perimeter, defining a proximal cavity.

The proximal cavity is located at the second end or proximal end of the medical fastening device, and, like any cavity, it comprises a bottom and an outer opening defining said volume. Therefore, the bottom and the opening of the cavity are separated from one another by a given distance.

The bottom of the proximal cavity defines the junction between the second end of the first through conduit and the second end of the second fastening conduit. In a particular embodiment, the annular proximal surface and the proximal cavity are located around the second longitudinal axis of the second fastening conduit intended for housing the first fastening element.

The annular proximal surface comprises two surfaces: an outer face intended for being in contact with the bone material and an inner face intended for being in contact with the graft. The proximal cavity can be considered a prolongation of the first through conduit and the second fastening conduit, such that it emerges from the second fastening element like a protrusion parting from the first and second longitudinal axes.

Therefore, in this embodiment, the annular fastening passage is the space or volume that is defined between the first fastening element and the annular proximal surface, when the first fastening element is housed in the second fastening conduit, such that it allows fastening the at least one graft.

Advantageously, this embodiment allows the proximal cavity to be part of the annular fastening passage, substantially and advantageously increasing the fastening surface. Accordingly, the fastening surface and the annular fastening passage of the fastening device of the present invention reinforce the fastening and the possibilities of the graft resulting in a successful repair. This new configuration provides the graft with an annular fastening surface sufficient for accommodating it as it expands when it is restrained.

This allows increasing the fastening force in the at least one graft and obtaining optimal fastening, thus solving the problems observed in the state of the art. This differs from what occurs with the fastening devices on the angled proximal end of an interference screw, in which since a very small or reduced fastening surface is available due to the area occupied by the screw itself, the fastening that is achieved may be incomplete.

Advantageously, the annular fastening passage of the present invention provides complete fastening of the graft, which prevents part of the material of the graft from coming out of the annular fastening passage as the graft is being restrained, thereby solving a problem observed in the state of the art. This differs from what occurs with other fastening devices, in which the fastening is sometimes incomplete as the fastening surface is very small or reduced.

In a particular embodiment, the second fastening element is a fastening ferrule.

In a particular embodiment, *the second fastening element comprises a distal appendage with a first end and a second end.*

In another particular embodiment, *the second end of the distal appendage is connected to the first end of the first through conduit, the distal appendage projecting from the first end of the first through conduit.* Preferably, the distal appendage is connected to the first end of the first through conduit.

In a particular embodiment, *the second end of the distal appendage is additionally housed in the first through conduit dividing said first through conduit into two sections, and the first end projects from the first end of the first through conduit.*

In another additional embodiment, the first through conduit has axial symmetry. On the other hand, the distal appendage is located on a first plane perpendicular to the entrance section of the first end of the first through conduit. This position shall be considered throughout the document as the distal appendage being in the vertical position.

In another embodiment, the distal appendage is located on a second plane perpendicular to the entrance section of the first end of the first through conduit, which in turn is also perpendicular to the first plane perpendicular to the entrance section of the first end of the first through conduit defined in the preceding embodiment. This position shall be considered throughout the document as the distal appendage being in the horizontal position.

The distal appendage allows keeping the branches of the at least one graft separated. Advantageously, these embodiments allow making it possible, in anatomical repairs of the cruciate ligaments by means of a single tibial bone tunnel, to provide the intra-articular segment of the branches of the graft with the precise degrees of twisting that the original ACL has in the specific flexion-extension position of the knee in which it is repaired. It must be understood that the intra-articular segment refers to the segment of the graft between both insertions, tibial and femoral, in the intra-articular cavity. It therefore corresponds to the entire part of the graft joining both bones, tibial and femoral, and located outside the bone tunnels, i.e., where the ACL is originally located.

In ACL repairs by means of a single tibial bone tunnel, the distal appendage advantageously allows leading into the tibial bone tunnel the two main branches of the ACL, the anteromedial (AM) branch and posterolateral (PL) branch, to the positions corresponding to both branches of the original insertions. Accordingly, the original anatomy of the ACL is advantageously restored.

Advantageously, in one or more particular embodiments, in which the distal appendage keeps the branches of the graft separated a certain distance, it allows more anatomical ACL repairs, in which this separation allows restoring the ample areas of insertion of the original ACL, both in the tibia and in the femur, without needing to use double tunnel techniques in the tibia and/or femur.

In one embodiment, *the first end of the distal appendage additionally comprises a hole, said hole being intended for the suspension of a bent end of the graft, such that when the first fastening element is housed and fastened in the second fastening conduit of the second fastening element, the medical fastening device allows fastening at least one free end of the graft while simultaneously at the same time the hole allows maintaining the suspension of the at least one bent end of the graft.*

The distal appendage with a hole is a suspension element configured for receiving the bent end of the graft, since it allows suspending the graft when it is introduced through the hole. This configuration allows the free ends of the graft to come back around so they can be fastened. In cruciate ligament repairs by means of a single graft of sufficient length, this advantageously allows the use of the 3-strand or 4-strand graft configuration without using sutures or knots. Preventing the use of sutures and knots allows both free ends of the graft to be fastened by the device in a direct mechanical manner.

Advantageously, this configuration reduces the size of the device since even though the intra-articular segment of the graft is in a 3-strand or 4-strand configuration, the device only has to fasten its two free ends once they have come back around from the path through the intra-articular region, from the tibia to the femur and, back around, from the femur to the tibia.

Therefore, in the 3-strand configuration, with a single tibial bone tunnel, the graft is configured with two loops, a tibial loop and a femoral loop, which are suspended from respective holes of the respective tibial and femoral fastening devices, with the free end of the graft coming from the tibial hole being fastened by the femoral fastening device and the free end of the graft coming from the femoral hole being fastened by the tibial fastening device.

In one embodiment, *the distal appendage is preferably a strip, cord, or band, wherein the first end of the distal appendage is connected to the second fastening element, and wherein the second end of the distal appendage is a free end, such that when the first fastening element is housed and fastened in the fastening conduit, additionally the second end of the distal appendage is housed in the fastening passage, configuring an adjustable loop, with the adjustable loop being intended for the suspension of a bent end of the graft.*

Advantageously, unlike cortical buttons this adjustable loop does not require knots and can be revised, such that where necessary, the first fastening element can be loosened to change the tension of the graft the bent end of which is suspended from the adjustable loop.

In a particular manner, the distal appendage in the form of a strip, cord, or band of the preceding embodiment is intertwined or interwoven.

In a particular embodiment, *the distal appendage is preferably a strip, cord, or band, in which the first end and the second end of the distal appendage are connected to the second fastening element, configuring a hole, the hole being intended for the suspension of a bent end of the graft, such that when the first fastening element is housed and fastened in the second fastening conduit of the second fastening element, the medical fastening device allows fastening at least one free end of the graft while simultaneously at the same time the hole maintains the suspension of the at least one bent end of the graft.* Since both ends of the distal appendage are connected, the distal appendage is fastened and non-adjustable.

In a particular manner, the distal appendage in the form of a strip, cord, or band of the preceding embodiment is intertwined or interwoven.

In a particular embodiment, *the first fastening element is a screw with a screw shaft and a head, and a washer, and wherein*
- *the head of the screw comprises a circular step concentric to the shaft of the screw, and*
- *the washer comprises an inner circular step with a reciprocal shape with respect to the circular step concentric to the shaft of the screw, wherein said inner circular step is located in the area of the washer for receiving the head of the screw.*

The inner surface of the washer is the inner face or section which is intended for housing the screw and it is where said circular step is located. On the other hand, the outer surface of the washer is the outer face of the washer intended for coming into contact with the graft and/or with the annular proximal surface.

Advantageously, this embodiment prevents the washer from being dragged to a position that is not coaxial with the shaft of the screw, which prevents the rotational movement of the screw from being transmitted to the washer, facilitating the insertion and fastening in the medical fastening device of the present invention.

In a particular embodiment, the first fastening element comprises
- a screw with a circular head and a threading on its shank, and
- a circular washer configured for being housed in the head of the screw, and
wherein the second fastening conduit comprises a threading complementary to the threading of the screw configured for fastening the screw to the second conduit.

The washer comprises an orifice and an inner surface extending from the orifice to an outer edge where said surface ends. The orifice is configured for receiving the shank of the screw and the inner surface of the washer is configured for housing the head of the screw. In the embodiments comprising a washer, the fastening surface is configured between the outer surface of the washer and the inner face of the annular proximal surface.

In these embodiments, as a result of the annular proximal surface being configured around the threaded shaft of the screw, since it completely surrounds the entire opening of the proximal cavity, the fastening device of the invention allows applying greater compressive force without dragging the graft, and without the fastening screw going through the graft during fastening. Accordingly, the present invention prevents the screw from necessarily going through and deteriorating the graft as occurs with current devices by means of a screw with a washer.

Advantageously, the graft is placed between a wide sector of the external face of the washer, and the inner face of the annular proximal surface. The device of the present invention provides a higher fastening stiffness than that which was provided by means of screw and washer devices of the state of the art.

In a particular embodiment, *the second fastening element comprises a movable junction area movable between the first through conduit and the second fastening conduit, and wherein said movable junction area is configured for:*
- *moving to a first position inside the second fastening conduit, and*
- *moving to a second position inside the first through conduit.*

Junction area must be understood as the area where the edges of the second end of the first through conduit and the second end of the second fastening conduit are joined.

On one hand, the first position leaves room for housing the graft. On the other hand, the second position protects the graft, by preventing it from being pinched and/or perforated by the first fastening element while it is being inserted in the second fastening conduit.

In a particular embodiment, the second end of the second fastening element additionally comprises at least one flange located on an axis perpendicular to the second longitudinal axis. Preferably, the at least one flange is in the form of an annular retaining lobe.

The at least one flange is configured for abutting with the cortical bone which demarcates the external upper part of the inlet opening into the bone tunnel. Advantageously, this embodiment prevents the movement of the medical fastening device of the present invention in the bone tunnel.

In a particular embodiment, the angle (α) is comprised between 30° and 60°.

In a preferred embodiment, the second end of the second fastening element comprises two flanges located on an axis perpendicular to the second longitudinal axis.

In a particular embodiment with an adjustable loop, the second fastening element comprises a strip or band additionally comprising grooves that are reciprocal to grooves existing in the first fastening element, preferably a screw without a washer. Advantageously, this embodiment reinforces the fastening of the adjustable loop configuring the device.

In one embodiment, the second fastening element is one-piece.

In one embodiment, the elements of the medical fastening device are manufactured from a semicrystalline and biocompatible thermoplastic polymer material.

In a particular embodiment, the first fastening element is manufactured from titanium, and the second fastening element is manufactured from polyether ether ketone.

In a particular embodiment, the second fastening element is two-piece, with a first part being manufactured from polyether ether ketone and to which there is coupled by means of clipping and/or ultrasounds, a second part comprising an interwoven or intertwined cord or band.

In another particular embodiment, the second fastening element is two-piece, manufactured from polyether ether ketone and with a titanium ring or half-ring.

In a particular embodiment, the second fastening element comprises at least one longitudinal or helicoidal rib on its outer surface.

Advantageously, in the embodiments in which the second fastening element comprises at least one longitudinal rib on its outer surface, the turning of the second fastening element inside the bone tunnel as the fastening screw is being screwed in is prevented.

In a particular embodiment, the annular proximal surface comprises at least one slot or groove on its inner face. In another embodiment, the annular proximal surface comprises at least one rib or projection on its inner face. In another embodiment, the annular proximal surface comprises at least one groove and at least one rib on its inner face.

Advantageously, in the embodiments in which the inner face of the fastening surface comprises at least one groove and/or rib, the fastening on the graft is reinforced.

In a second inventive aspect, the invention provides a *system for inserting a medical fastening device, comprising*
- *a medical fastening device according to any of the embodiments of the first inventive aspect,*
- *an inserter, comprising*
   ∘ *a first end with a reciprocal shape with respect to the shape of the second fastening element and configured for engaging the second end of the second fastening element,*
   ∘ *a handle located at a second end of the inserter, and*
- *a coupling screw.*

In particular, the inserter system of the present invention allows providing a fast and efficient system for implanting a medical fastening device in a patient.

In a further example, there is provided *a medical fastening device for the fastening of a graft, wherein the medical fastening device is suitable for being inserted in a bone tunnel of a bone, and wherein said medical fastening device comprises*
- a first fastening element,
- a second fastening element, comprising:
   ∘ a fastening conduit, intended for housing the first fastening element configuring a fastening passage for fastening either against the bone or else against the device itself, and
   ∘ a distal appendage configuring a suspension element, preferably hole or loop, intended for the suspension of at least one bent end of the graft.

It must be understood that the definition of graft indicated in the first inventive aspect applies *mutatis mutandis* in all the embodiments of the third inventive aspect.

The hole or loop is configured for receiving and suspending a bent end of the graft. This configuration allows the free ends of the graft to come back around so that they can be fastened. This advantageously allows using in cruciate ligament repairs by means of a single graft of sufficient length, a 3-strand or 4-strand graft configuration without the use of sutures or knots. Preventing the use of sutures and knots allows both free ends of the graft to be fastened by the device in a direct mechanical manner.

Furthermore, this configuration advantageously reduces the size of the device since when the intra-articular segment has a 3-strand graft configuration, the graft is configured with two loops, a tibial loop and another femoral loop, which are suspended from respective holes or loops of the respective tibial and femoral fastening devices, with the free end of the graft coming out from the tibial hole or loop being fastened by the femoral fastening device and the free end of the graft coming from the hole or loop located in the femoral bone tunnel being fastened by the tibial fastening device.

Similarly, in the 4-strand configuration, the device only has to fasten its two free ends once they have come back from the path through the intra-articular region, from the tibia to the femur and, back around, from the femur to the tibia.

In a particular embodiment, the distal appendage is preferably a strip, cord, or band and comprises a first end and a second end, wherein the first end of the distal appendage is connected to the second fastening element, and wherein the second end of the distal appendage is a free end, such that when the first fastening element is housed and fastened in the fastening conduit, additionally the second end of the distal appendage is housed in the fastening passage, configuring an adjustable loop, with the adjustable loop being intended for the suspension of a bent end of the graft.

In a particular manner, the distal appendage in the form of a strip, cord, or band of the preceding embodiment is intertwined or interwoven.

Advantageously, in one or more particular embodiments in which the hole or loop keeps the branches of the bent end of the suspended graft separated a certain distance, allowing more anatomical ACL repairs, in which this separation allows restoring the ample areas of insertion of the original ACL, both in the tibia and in the femur, without needing to use double tunnel techniques in the tibia and/or femur.

In a particular embodiment, the distal appendage is preferably a strip, cord, or band and comprises a first end and a second end, wherein the first end and the second end of the distal appendage are connected to the second fastening element, configuring a hole or loop, the hole or loop being intended for the suspension of a bent end of the graft, such that when the first fastening element is housed and fastened in the fastening conduit, the medical fastening device allows fastening at least one free end of the graft in the fastening passage, while simultaneously at the same time the hole or loop allows maintaining the suspension of the at least one bent end of the graft.

In a particular manner, the distal appendage in the form of a strip, cord, or band of the preceding embodiment is intertwined or interwoven.

All the features of the embodiments described for the first inventive aspect apply to this third inventive aspect, with the exception of those exclusive or incompatible combinations.

In the same manner, all the features described in this specification (including the claims, description, and drawings) can generally be combined in any combination, with the exception of combinations of such mutually exclusive features.

### Description of Drawings

These and other features and advantages of the invention will become more apparent from the following detailed description of preferred embodiments given solely by way of nonlimiting illustrative examples in reference to the attached drawings.
Figures 1A to 1E show several graft retaining systems of the state of the art.
Figures 2A to 2E show a graft fastening system of the state of the art where the retention is performed in the upper part of the inside of the ferrule.
Figures 3A to 3D show several exploded views of the medical fastening device of the first inventive aspect.
Figures 4A to 4C show several views of the medical fastening device of the first inventive aspect, when the first fastening element is housed in the second fastening element.
Figures 5A and 5B show several views of the medical fastening device of the first inventive aspect with the graft being fastened.
Figures 6A to 6D show several views of the medical fastening device of the first inventive aspect with a distal appendage configuring an adjustable loop.
Figures 7A to 7C show several views of the medical fastening device of the first inventive aspect with a distal appendage configuring a fastened loop.
Figures 8A to 8C show several views of the medical fastening device of the first inventive aspect with a distal appendage configuring a half-ring.
Figures 9A to 9D show several views of the medical fastening device of the first inventive aspect with a horizontal distal appendage.
Figures 10A to 10D show several views of the medical fastening device of the first inventive aspect with a vertical distal appendage.
Figures 11A to 11E show several views of the system for inserting a medical fastening device according to the first and second inventive aspects.
Figures 12A to 12D show the steps for using a medical fastening device according to the first inventive aspect, with an adjustable loop in the femur, and a vertical appendage without a hole, during right knee ACL repair.
Figures 13A to 13D show a schematic view of a system of devices of the first inventive aspect as a whole, which allow the 3-strand configuration of a single graft.
Figures 14A to 14C show a schematic view of a system of devices of the first inventive aspect as a whole, which allow the 4-strand configuration of a single graft.
Figures 15A to 16D show several views of the device of the third inventive aspect.
Figures 17A to 17D show several views of the medical fastening device of the first inventive aspect with two flanges located at the second end of the second fastening element.
Figures 18A to 18D show some steps of a surgical method for restoring a damaged anterior cruciate ligament (ACL) by means of a first and a second fastening devices of the invention.

### Detailed Description of the Invention

The present invention describes two alternative graft fastening devices. Preferably, in the embodiments described in this section the second fastening element can be one-piece, manufactured from a semicrystalline and biocompatible thermoplastic polymer material; the first fastening element may comprise a screw made of a titanium alloy and a washer made of a semicrystalline and biocompatible thermoplastic polymer material, or of any other material described in the state of the art. Additionally, the outer surface of the second fastening element may comprise grooves which favor osseointegration of the device by increasing the contact surface with the bone.

All possible geometries, all possible materials, all possible methods of manufacture, and all possible surface treatments to be found in the medical literature are contemplated in other embodiments for the second fastening element and for the first fastening element.

The first fastening element being one-piece is likewise contemplated in other embodiments.

In the repair of the anterior cruciate ligament (ACL) of the knee by means of grafts, a femoral bone tunnel is normally used in which the bent end of the graft is suspended by means of fastening devices described herein, with loop, adjustable loop, hole, or suspension element. On the other hand, one or more fastening devices described herein are used in one or two tibial tunnels having a diameter of between 6 mm and 12 mm for fastening the other end of the graft. Therefore, this range is the preferred size envisaged for the second fastening elements of the medical fastening devices intended for being used in the embodiments shown.

This must not limit the applications of the present invention, which works efficiently in the repair of any ligament present in a joint, with suitable dimensions in each case.

### First fastening device

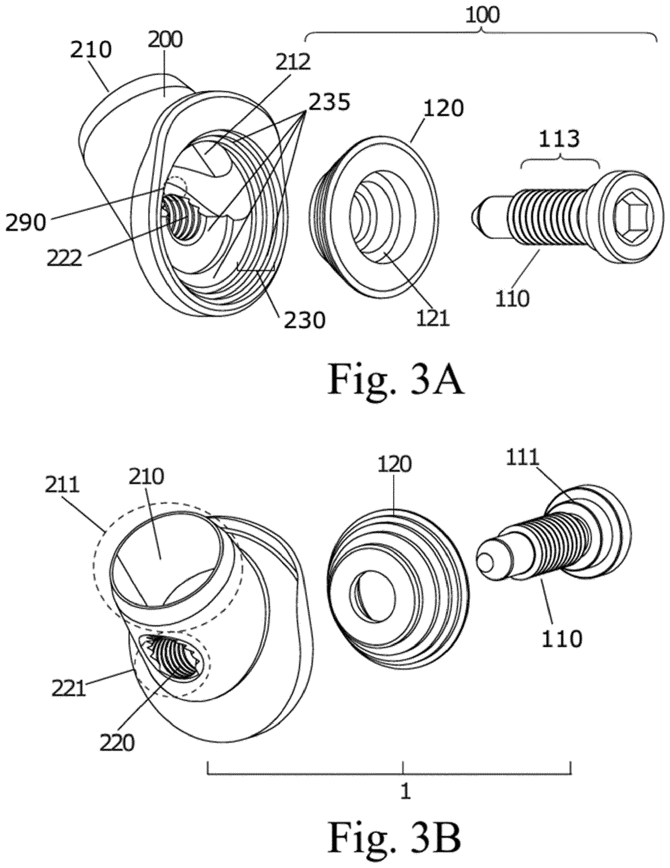

Figures 3A to 14D show embodiments of the first medical fastening device (1). In particular, the medical fastening device (1) comprises a first fastening element (100) and a second fastening element (200). It must be observed that in any of the described examples, the second fastening element (200) can be a fastening ferrule.

### First fastening device: first fastening element (100)

An embodiment of a first fastening element (100) according to the first invention can be observed in Figures 3A to 3D. In this example, the first fastening element is a fastening screw (110) with a head provided with an Allen-type actuation area and a threading (113) that is reciprocal with respect to the threading (223) of the second conduit (220).

Furthermore, the first fastening element (100) comprises a washer (120) surrounding the head of the screw when in use.

Figures 3A to 3D show a view of the screw (110) and the washer (120). In particular, the head of the screw is reciprocal with respect to the shape of the inner surface of the washer (120). In this embodiment, the screw (110) comprises a circular step (111), in a plane perpendicular to the shaft of the screw. In turn, there is provided in a reciprocal manner in the washer (120) an inner circular step (121) opposite that of the screw. Advantageously, this example prevents the engagement of the washer with the screw from seizing up, which allows keeping the washer aligned with the screw and optimizes the transmission of force between both.

### First fastening device: first embodiment of the second fastening element (200) : Figures 3A to 5B

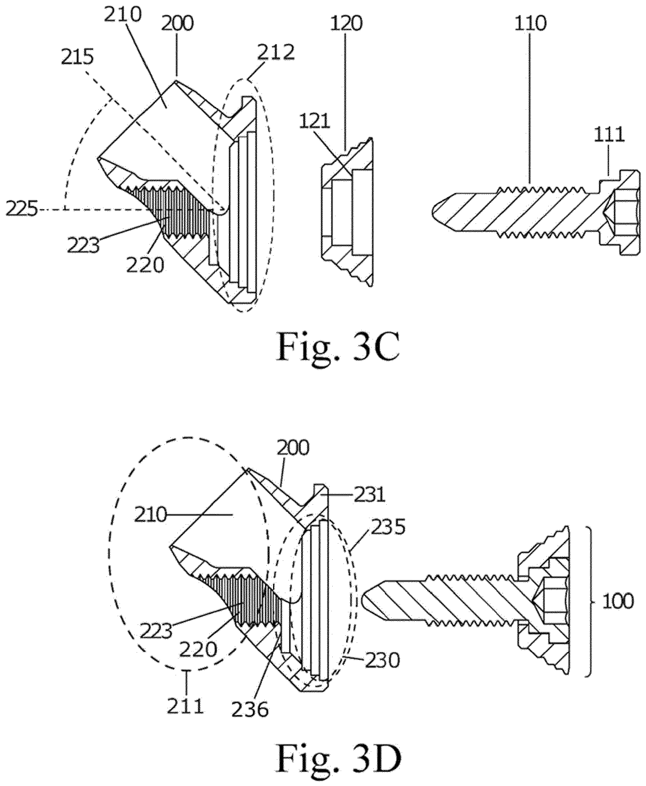

Figures 3A to 5B show a first embodiment of the first medical fastening device (1). In particular, the second fastening element (200) comprises a first through conduit (210) which is extended along a first longitudinal axis (215). As can be seen in the section view of Figures 3C and 3D, the second fastening element (200) further comprises a first end (211) and a second end (212).

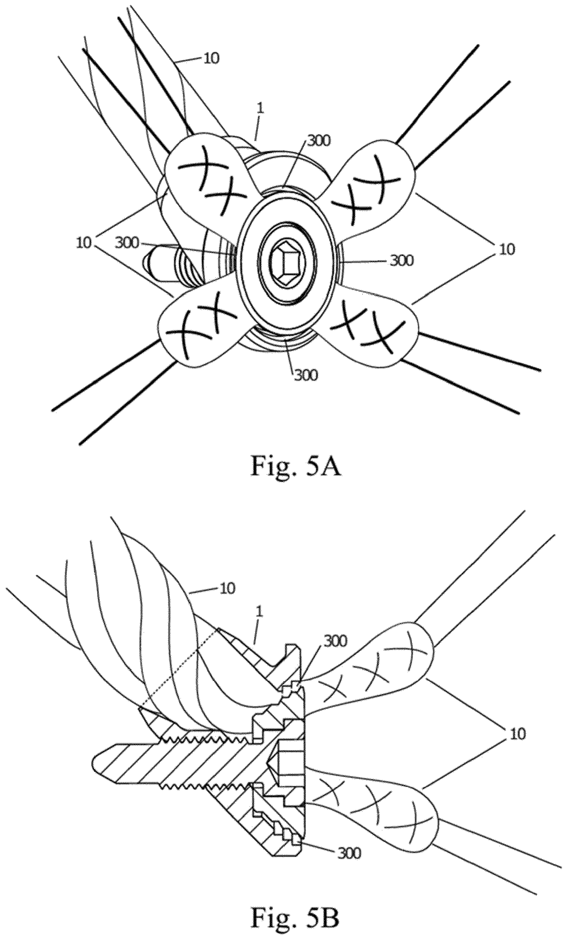

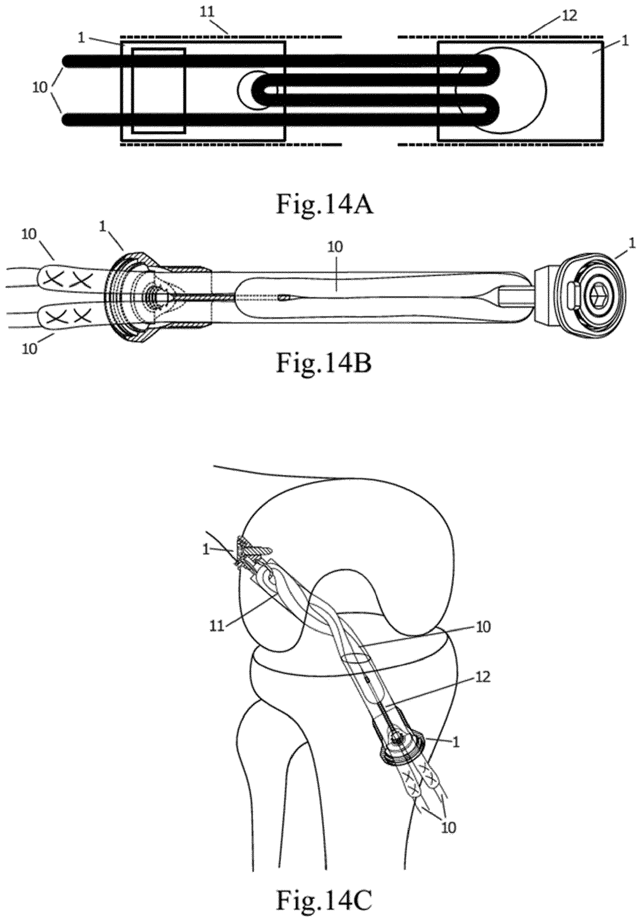

In relation to the first through conduit (210) of the second fastening element (200), Figures 3A and 3B show that said through conduit (210) further comprises a first end (211) and a second end (212). In particular, the first through conduit (210) is configured for housing a graft (10) as will be shown below in the description of the method of use indicated in Figures 5A to 5B, 14A to 14D. In particular, the first end (211) is intended for receiving the graft (10), the second end (212) is intended for being part of the fastening surface of the annular fastening passage (300) as shown in Figures 5A and 5B.

The configuration of the distal end of the first conduit (210) of the second fastening element (200) can be circular, elliptical, rectangular, trilobular, half-annular, or correspond to any other geometric shape known in the state of the art, without there necessarily having to be a correspondence between the configuration of the distal end and of the proximal end.

Furthermore, as can be seen in Figures 3A to 3D, the second fastening element (200) comprises a second fastening conduit (220) which is extended along a second longitudinal axis (225). The second fastening conduit (220) further comprises a first end (221) and a second end (222).

The second fastening conduit (220) is configured for housing and fastening the first fastening element (100) as can be seen in Figures 3A to 5B. To that end, the second fastening conduit (220) can have several forms on its inner face according to the form of the type of the first fastening element (100). For example, in the section view of Figure 3C, the inner face of the second fastening conduit (220) has the form of threading complementary to the threads of a screw. In another embodiment, the inner face of the second conduit (220) is in the form of a clip or leaf spring complementary to the outer surface of the first fastening element (100). In another embodiment, the inner face of the second fastening conduit (220) has a smooth form for a first self-tapping fastening element (100).

As can be seen in the example of the section view of Figure 3C, the first through conduit (210) and the second fastening conduit (220) are arranged inside the second fastening element (200) such that the first longitudinal axis (215) and the second longitudinal axis (225) define an angle (α) with one another other than 0, preferably an angle between 0° and 90°. Preferably, the angle (α) is comprised between 30° and 60°. Even more preferably, in these embodiments the angle (α) can be 45°.

In particular, Figure 3A shows that the second fastening element (200) comprises an annular proximal surface (230) which defines a proximal cavity (235) at the second end (212) of the second fastening element (200). Furthermore, the second end (212) of the first through conduit (210) and the second end (222) of the second fastening conduit (220) are joined defining a junction (290) at the bottom of the proximal cavity (235). The junction (290), the second end (212) of the first through conduit (210), and the second end (222) of the second fastening conduit (220) are part of the bottom of the proximal cavity (235) of the second end (212) of the second fastening element (200) extending to the opening of said proximal cavity (235).

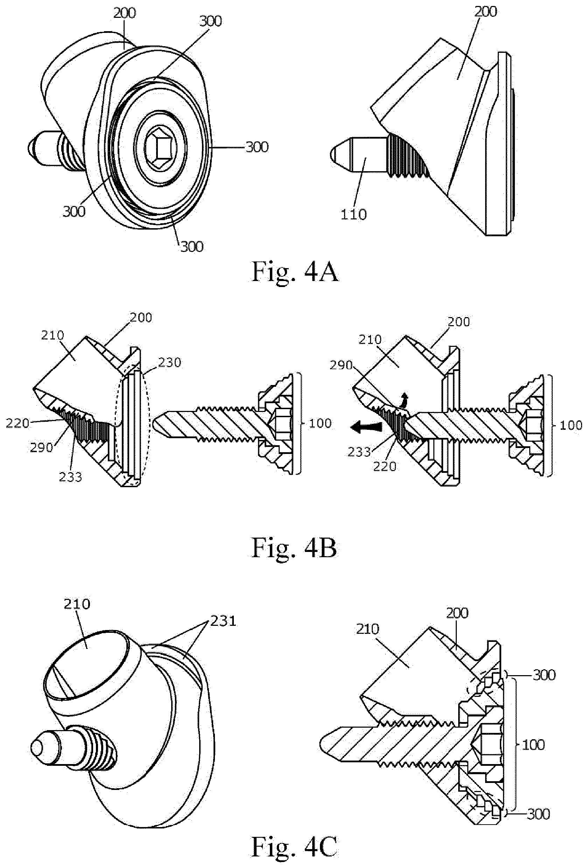

Furthermore, it can be seen in Figure 4B that the junction (290) is movable in this embodiment.

As can be seen in the embodiment of Figure 3D, there is a lower stop (236) on the annular proximal surface (230) of the second fastening element (200). Therefore, the annular proximal surface (230) comprises a lower stop (236). The combination of the annular proximal surface (230) and stop (236) allows there to be a position of maximum tightness, in which a sector of the contour of the first fastening element (100) is supported on the lower stop (236), which advantageously increases the rigidity of the fastening provided by the device.

The stop (236) allows the fastening of the graft to a predetermined thickness, providing rigidity to the fastening. The annular configuration of the annular proximal surface (230) guarantees enough space for expansion of the graft (10) from the starting thickness to this predetermined thickness which corresponds with the position of maximum tightness of the fastening device (1).

As can be seen in the embodiment of Figures 3A to 5B, the second fastening element comprises a flange (231) which advantageously prevents migration of the device (1) into the bone tunnel.

Finally, Figures 4A to 5B show an embodiment with the first fastening element (100) housed and fastened in the second fastening conduit (220) of the first embodiment of the second fastening element (200). It can be seen how the annular fastening passage (300) is formed between the inner face of the annular proximal surface (230) and the outer surface of the washer (120).

Figures 5A and 5B show views of the upper part as a whole and the lower part in section, respectively, of the medical fastening device with at least one graft (10) restrained therein. In particular, the annular fastening passage (300) created by the medical fastening device (1) of the present invention can clearly be seen in Figure 5B. Said annular fastening passage (300) is annular because the arrangement of the annular proximal surface (230) generates the continuous and annular surface of the proximal cavity (235). This allows the annular proximal surface (230) to be part of the annular fastening passage (300), which allows fastening several grafts with a larger fastening passage than in the elements mentioned of the state of the art. Furthermore, the shape of the annular fastening passage (300) provides greater structural consistency to the fastening.

If Figures 5A and 5B are compared with Figures 1A to 1F, the differences of the medical fastening device (1) of the present invention with respect to other devices (600, 700, 800) of the state of the art can clearly be seen. The interference screw (600) of Figures 1A and 1B performs fastening against the bone, where it is a very weak fastening due to the porosity of said bone (900). Furthermore, it can be seen that said interference screw (600) can damage the ligament, even further weakening the fastening.

The screw (700) of Figures 1C and 1D is coaxially screwed into the ferrule housing the graft, directly onto the graft, which, like the interference screw, may damage said graft. Furthermore, in the case of use in the tibia, the largest part of the head of the screw is housed inside the tibia bone which, due to the high porosity of this bone, weakens the resistance to movement of the device.

The restraining element (800) of Figures 1E to 1F is introduced into the bone tunnel and restrains the graft against the internal wall of the tunnel, while at the same time the screw (802), with the washer, which enters perpendicular to the cortical bone and is screwed into the angled face of the restraining element, restrains the graft a second time. This configuration locates the fastening area around the screw with the washer, so the screw must necessarily separate and pass between the bundles of the graft to proceed to the fastening thereof, which, like in the two prior art examples, may damage the graft, in addition to causing other problems associated with these devices that have been described in the background of the invention.

In contrast, the first medical fastening device (1) prevents damaging the ligament with the screw (110) due to the fact that the first conduit (210) and the second conduit (220) are arranged inside the second fastening element (200) such that the first longitudinal axis (215) and the second longitudinal axis (225) define an angle (α) with one another other than 0. Furthermore, like in the first medical fastening device (1) the ligament is housed inside same, with the aid of the annular proximal surface (230), a larger fastening passage (300) is obtained and fastening against porous bone (900) is prevented, providing a stronger and more durable fastening.

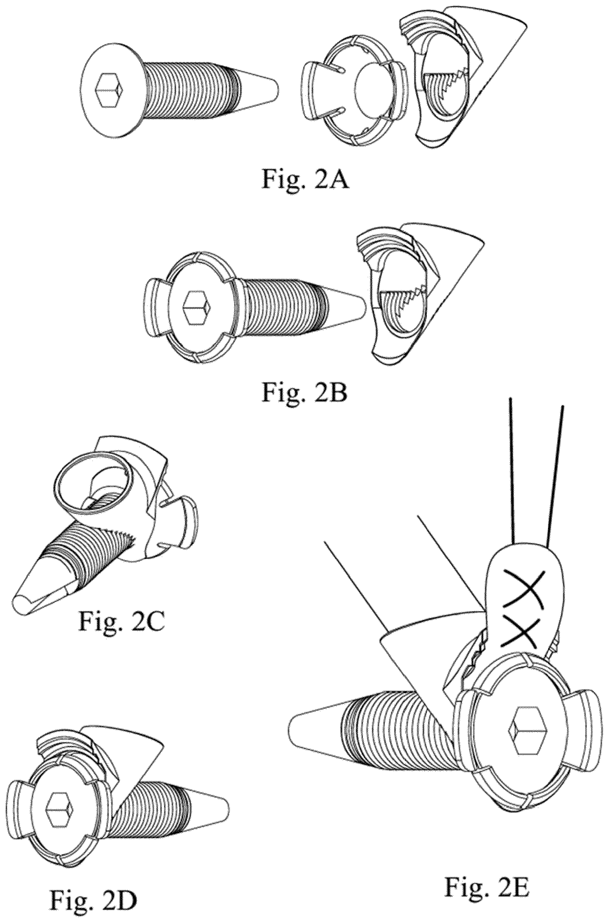

The configuration of the fastening passage exclusively in the upper proximal area of the ferrule can be seen in Figure 2E. If Figure 2E is compared with Figures 5A and 5B, Figure 2E provides a limited fastening passage which leaves part of the graft (10) unrestrained, which reduces both the stability of the fastening and the ultimate tensile strength offered by the device, unless the device is oversized, which is not an acceptable solution.

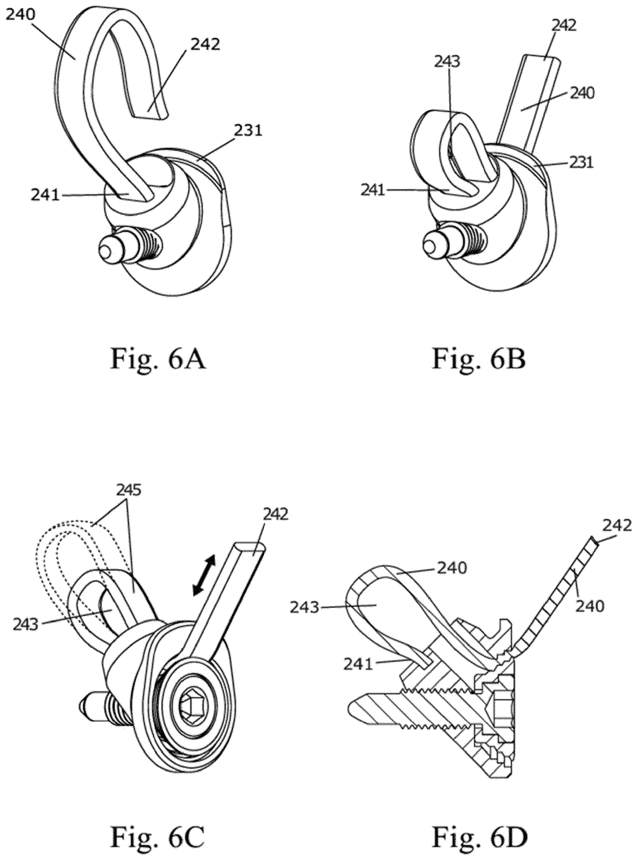

### First fastening device: second embodiment of the second fastening element (200): distal appendage (240) configuring an adjustable loop (245); Figures 6A to 6D

Figures 6A to 6D show a second embodiment of the first medical fastening device (1) of the invention. As can be seen, the second embodiment is similar to the first embodiment, with the exception that the second embodiment further comprises a distal appendage (240) configuring an adjustable loop (245).

As can be seen in Figure 6A, the distal appendage (240) comprises a strip, cord, or band, with a first end (241) joined to the first end of the second fastening element (200) and a second end (242) with a first fastening-free position. In a second position, the second end (242) can be configured such that said end (242) goes back to the second fastening element (200) configuring an adjustable loop (245) which is fastened in the desired position by means of the first fastening element (100), as shown in Figures 6B, 6C, and 6D.

In particular, as shown in Figures 6B to 6D, once the first fastening element (100) is housed and fastened in the second fastening conduit (220) of the second fastening element (200), the free end (242) is also fastened in said fastening passage (300).

As can be seen in Figure 6C, the dotted line shows an example of the different positions the adjustable loop (245) can take; therefore, it can be considered that the distal appendage (240) is flexible and can define more than one final position in this embodiment.

The length of the adjustable loop (245) is fastened by means of the first fastening element (100), the adjustable loop (245) being intended for the suspension of a bent end of the graft (10) in the bone tunnel or bone canal. Said fastening is adjustable from a first position without tension, to a final position with ideal tension of the suspended graft.

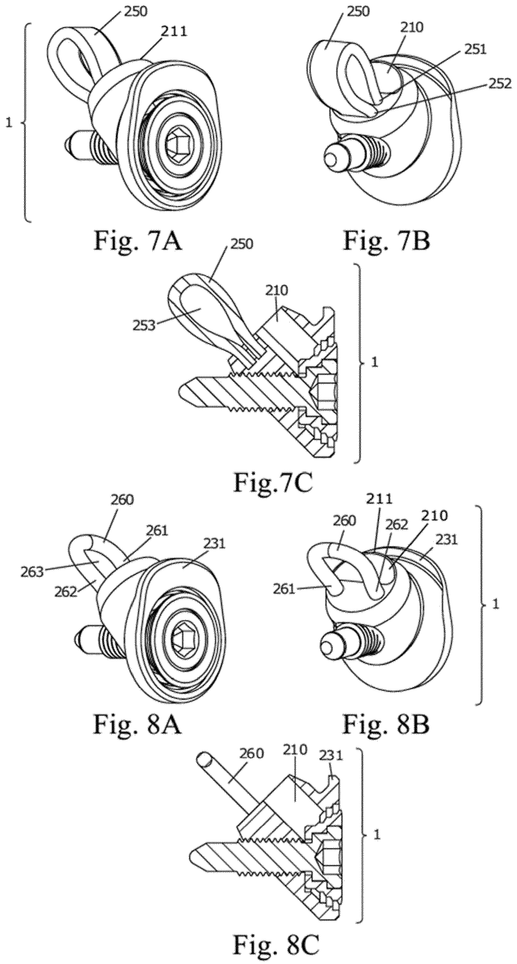

### First fastening device: third embodiment of the second fastening element (200): distal appendage (250) configuring a fastened loop; Figures 7A to 7C

Figures 7A to 7C show a third embodiment of the first medical fastening device (1) of the invention. As can be seen, the third embodiment is similar to the first embodiment, with the exception that the third embodiment further comprises a distal appendage (250) with hole (253) configuring a non-adjustable loop. It can be called a fastened or non-adjustable loop because both ends (251, 252) of the appendage (250) are fastened to the second fastening element (200), and therefore the area of suspension, i.e., the size of the hole (253), is constant. In one embodiment, the distal appendage (250) is flexible. In the examples of Figures 7A to 7C, the distal appendage (250) is in the vertical position.

As can be seen in this particular embodiment, the distal appendage (250) comprises a strip, cord, or band, with both ends (251, 252) joined to the second fastening element (200), such that a closed or non-adjustable loop intended for the suspension of a bent end of the graft in the bone tunnel is configured.

### First fastening device: fourth embodiment of the second fastening element (200): distal appendage (260, 270) with a hole for suspension; Figures 8A to 9D

Figures 8A to 8C, show a fourth embodiment of the first medical fastening device (1) of the invention. As can be seen, the fourth embodiment is similar to the first embodiment, except that the fourth embodiment further comprises a distal appendage (260). In these examples, the distal appendage (260) is a half-ring defining a hole (263), intended for the suspension of a bent end of the graft in the bone tunnel.

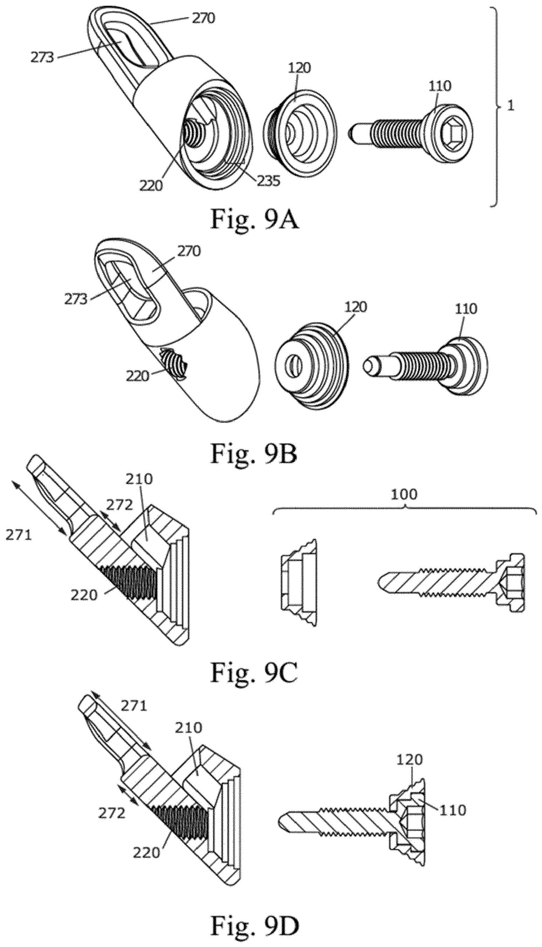

In the particular embodiment of Figures 9A to 9D, the second fastening element (200) does not comprise a flange (231), and instead it has a frustoconical external contour, which advantageously retains the device at the inlet of the bone tunnel (10), preventing it from penetrating the bone tunnel (10), with said second fastening element (200) being similar to that shown in the first embodiment of the first invention in terms of the remaining aspects.

As can be seen in the examples of Figures 9C and 9D, the distal appendage (270) comprises a first end (271) and a second end (272) located at the first end of the second fastening element (200). Furthermore, the distal appendage (270) comprises a hole (273) intended for the suspension of a bent end of the graft in the bone tunnel. More particularly, the hole (273) is located at the first the end (271) of the distal appendage (270).

In other embodiments, the second fastening element (200) of Figures 9A to 9D may comprise a flange (231) like the one shown in the examples of Figures 8A to 8D.

In view of Figures 8A to 9D, it can be considered that the distal appendage (260, 270) is in the horizontal position.

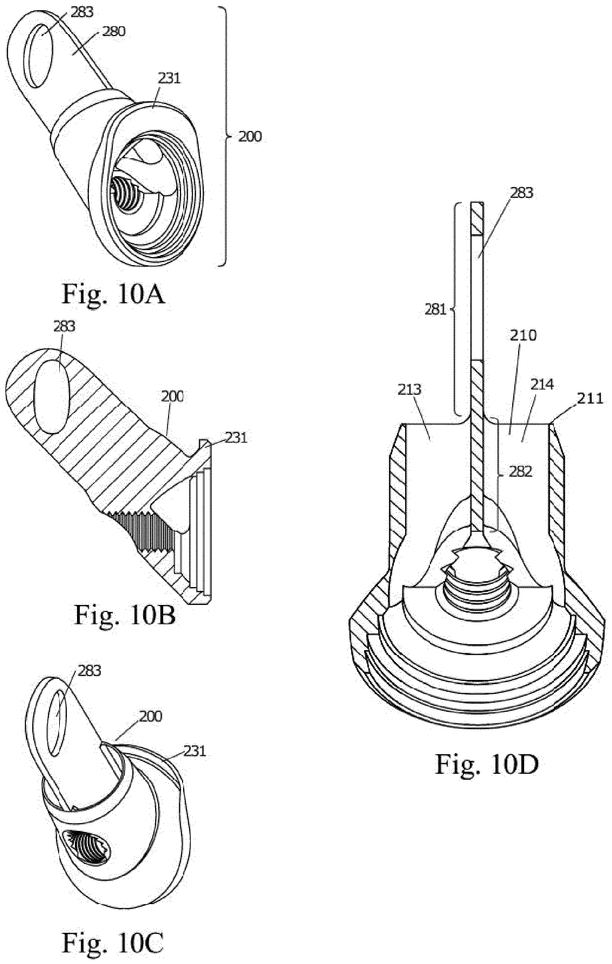

### First fastening device: fifth embodiment of the second fastening element (200): distal appendage (280) with a hole (281) for suspension; Figures 10A to 10D

Figures 10A to 10D show a fifth embodiment of the first medical fastening device (1) of the invention. As can be seen, the fifth embodiment is similar to the first embodiment, with the exception that the fifth example further comprises a distal appendage (280) located at the first end of the second fastening element (200). In these examples, the distal appendage (280) comprises a first end (281), a second end (282), and a hole (283) intended for the suspension of a bent end of the graft in the bone tunnel. Furthermore, the hole (283) is located at the first the end (281) of the distal appendage (280).

In view of Figures 10A to 10D, it can be considered that the distal appendage (280) is in vertical position.

In a particular manner, the second end (282) is housed in the first through conduit (210) dividing the first through conduit (210) into two sections. In other words, the first through conduit (210) is divided into a first section (213) and a second section (214) by the second end (282) of the distal appendage (280).

On the other hand, Figures 10A, 10B, and 10D show that the second end (282) of the distal appendage (280) is part of the bottom of the proximal cavity (235) and of the junction between the second end (212) of the first through conduit (210) and the second end (222) of the second fastening conduit (220).

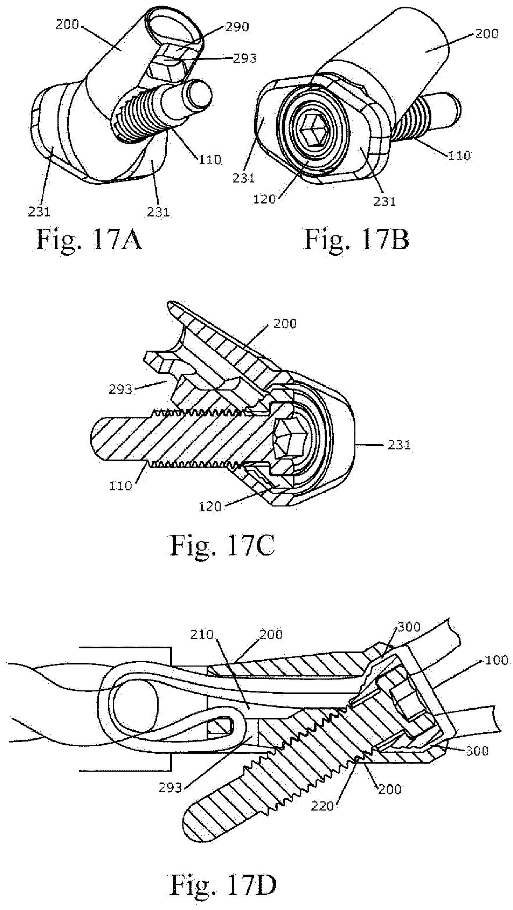

### First fastening device: sixth embodiment of the second fastening element (200): distal appendage (290) with a hole (293) for suspension; Figures 17A to 17D

Figures 17A to 17D show a sixth embodiment of the first medical fastening device (1) of the invention. As it can be seen, the sixth embodiment is similar to the first embodiment, with the exception that the second end (222) of the second fastening conduit (220) in the sixth example further comprises two flanges located opposite on an axis perpendicular to the second longitudinal axis (225). Another difference with the first embodiment is that the sixth example comprises a distal appendage (290) located at the first end of the second fastening element (200).

In the example, the two flanges (231) are in the form of an annular retaining lobe configured for abutting with the cortical bone which demarcates the external upper part of the inlet opening into the bone tunnel.

Advantageously, the flanges (231) retains the device at the inlet of the bone tunnel (10), preventing it from penetrating the bone tunnel (10).

In the example, the distal appendage (290) comprises a hole (293) intended for the suspension of a bent end of the graft in the bone tunnel.

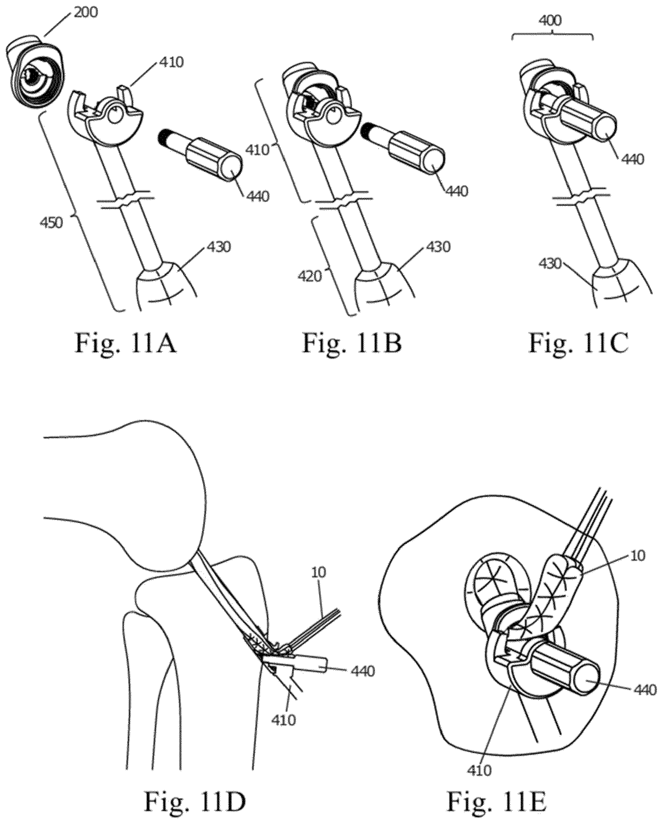

### First fastening device: system (400) for inserting a medical fastening device (1): Figures 11A to 11E

Figures 11A to 11E show an embodiment of a system (400) for inserting a medical fastening device (1) according to any of the embodiments shown above. The system (400) comprises the medical fastening device (1) of the present invention, a coupling screw (440), and an inserter (450) with a first end (410) configured for engaging the second end of the second fastening element (200) and a handle (430) located at a second end (420) of the inserter (450).

Advantageously, the use of an inserter (450) with coupling screw (440) allows handling the device through the handle (430) and applying the necessary force for the insertion of the device (1) in the bone tunnel (11). This prevents the device (1) from uncoupling from the inserter (450), as can be seen in greater detail in Figures 11A and 11E.

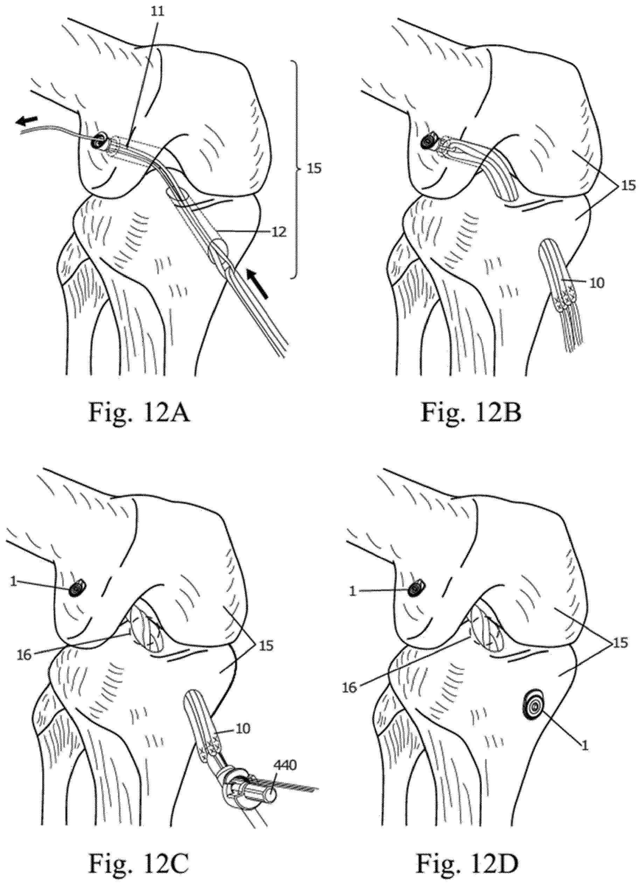

### First fastening device: example of use of a medical fastening device, with an adjustable loop in the femur and rigid vertical distal appendage in the tibia: Figures 12A to 12D

Figures 12A to 12D show the repair of an anterior cruciate ligament (ACL) of a right knee (15) using two of the first medical fastening devices (1) of the invention. The first medical fastening device (1) is similar to that described in the example of Figures 6A to 6C and the second medical device (1) comprises a distal appendage in the vertical position, without a hole.

Advantageously, this distal appendage without a hole allows maintaining the twisting of the intra-articular segment of the graft branches without said twisting reaching the intra-tunnel segment, which is important for restoring the anatomy and biomechanics of twisting of the original ACL, as can be seen in the embodiment of Figures 12A to 12D.

Figure 12A shows the free ends of the grafts (10) projecting from the bone tunnel (12) made in the tibia. The bent ends of the grafts (10) are suspended from the adjustable loop (245) of the first medical fastening device (1).

Advantageously, the adjustable loop (245) allows making a femoral bone tunnel (11), or a femoral bone canal, and pulling on the bent end of the graft to fit it in, which further allows checking and adjusting the tension at which the graft (10) is fastened.

Figure 12B shows the bents ends of the grafts once they are fitted in, with the adjustable loop being restrained by the femoral fastening device. The free ends of the grafts (10) still project from the bone tunnel (12) made in the tibia.

Figure 12C shows the twisting of the intra-articular segment of the graft (10) which mimics the anatomical twisting (16) characteristic of the original ACL, i.e., counter-clockwise direction, in the ACL of the right knee which is shown. Advantageously, the second medical device (1) with a distal appendage and without a hole shown in Figure 12C allows keeping the branches of the graft (10) separated, such that the anatomical twisting imparted to the intra-articular segment of the graft does not uncontrollably propagate into the bone tunnel. As can be seen, an inserter system (400) is used for handling the device.

Figure 12D shows the fastening device (1) with the first threaded fastening element (100) once the graft (10) has been tensed at the tension necessary for recovering the stability in the knee joint.

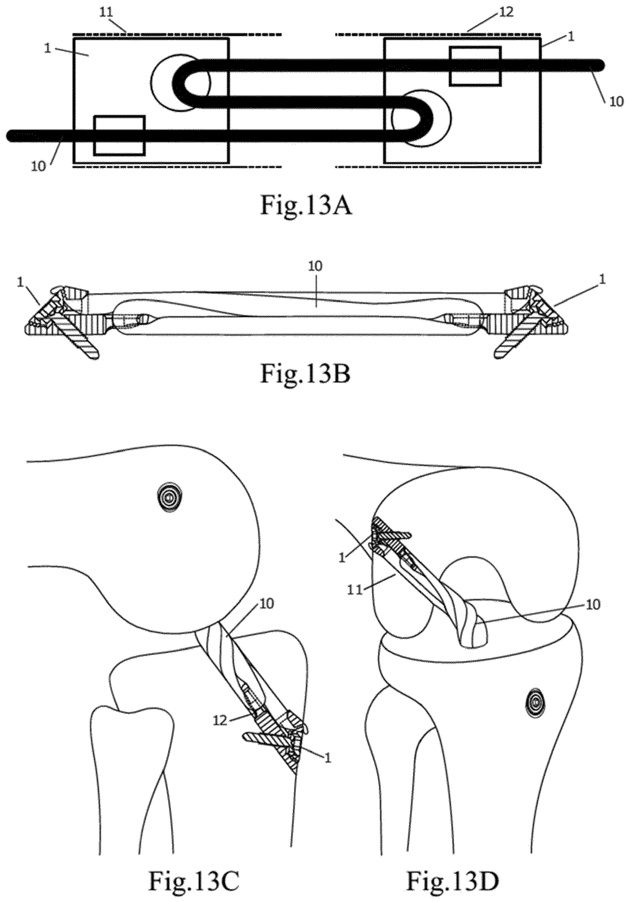

### First fastening device: example of use of the first medical fastening device (1) of the invention: 3-strand configuration of a graft: Figures 13A to 13D

Figure 13A shows a diagram of the joint use of two first medical devices (1) of the invention, in the repair of the ACL of the right knee, in which said use comprises:
- providing a graft (10) which is configured in three branches, with two bent ends and two free ends.
- providing two medical fastening devices (1) with a distal appendage (250, 260, 270, 280) with a hole, intended for being introduced in respective first and second bone tunnels (11, 12). The first fastening element (100) and second fastening element (200) assembly is depicted as a rectangle in Figure 13A. The hole (253, 263, 273, 283) is depicted as a circle in Figure 13A.
- configuring, as shown in Figures 13B to 13D, the two medical fastening devices (1) such that it allows fastening a first free end and suspending a first bent end of the graft (10) by means of the first medical device (1) inserted in the first bone tunnel (11). On the other hand, fastening a second free end and suspending a second bent end of the graft (10) by means of the second medical device (1) inserted in the second bone tunnel.

Figures 13B to 13D show the use of two medical fastening devices (1) such as those shown in Figures 9A to 9D for the 3-strand configuration of a single graft, according to the diagram of Figure 13A.

### First fastening device: example of use of the medical fastening device (1) of the first invention: 4-strand configuration of a graft: Figures 14A to 14C

Figure 14A shows the use diagram of two first medical devices (1) of the invention in the repair of the ACL of the right knee, in which said use comprises:
- providing a graft (10) with a 4-strand configuration in four branches, with three bent ends and two free ends.
- providing two medical fastening devices (1), a first device with a vertical distal appendage with a hole (280), of Figures 10A to 10D, intended for being introduced in a first bone tunnel (11), and a second device with an adjustable loop, intended for being introduced in a second bone tunnel (12). The first fastening element (100) and second fastening element (200) assembly is depicted as a rectangle in Figure 14A. The suspension element, i.e., hole or adjustable loop, is depicted as a circle in Figure 14A.
- configuring, as shown in Figures 14B and 14C, the two medical fastening devices (1) such that they allow fastening both free ends and simultaneously suspending a first bent end of the graft (10), by means of the first medical device (1) inserted in the first bone tunnel (11). On the other hand, by means of the second medical device (1) inserted in the second bone tunnel (12), suspending, in an adjustable manner, a second and a third bent end of the graft (10).

### Second fastening device

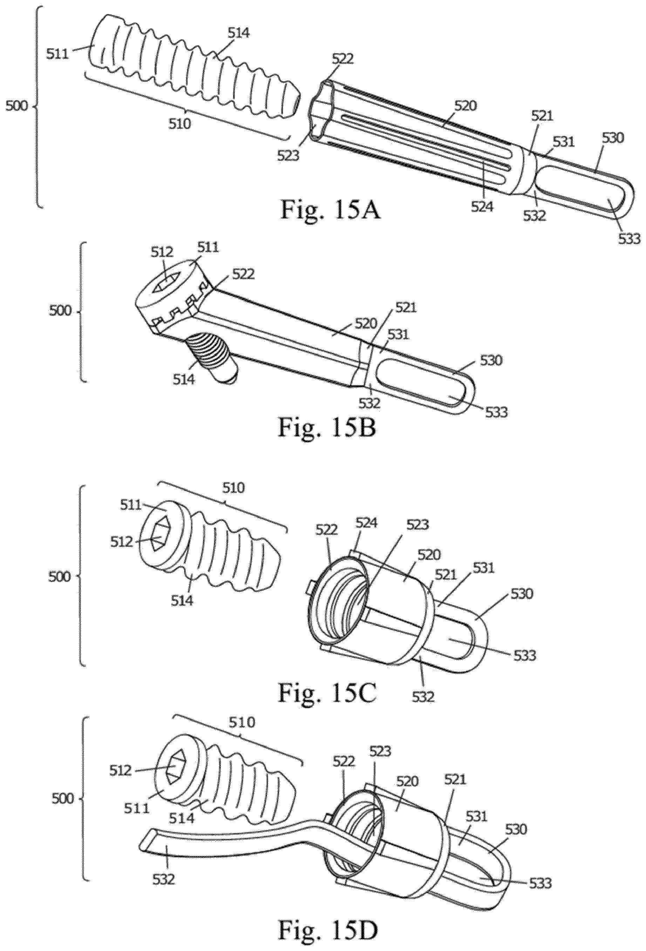

Figures 15A to 16D show embodiments of the second medical fastening device (500) of the invention. In particular, the medical fastening device (500) comprises a first fastening element (510) and a second fastening element (520) comprising a fastening conduit (523) and a suspension element. In this second fastening device (500), once the first fastening element (510) is fastened in the fastening conduit (523) of the second fastening element (520) and introduced in the bone tunnel, a fastening passage is produced between the second fastening element (520) and the bone tunnel which fastens the graft in said bone tunnel, or between the first fastening element (510) and the second fastening element (520) which fastens the graft to the device itself.

### Second fastening device: first fastening element (510)

An embodiment of a first fastening element (510) according to the second fastening device can be observed in Figures 15A to 16D. In the examples of Figures 15A, 15C, and 15D, the first fastening element (510) is a screw the head of which has the same diameter as its shank (514). In the example of Figure 15B, the diameter of the head of the screw is greater than the diameter of its shank (514). The head of the screw (511) is provided with an Allen-type actuation area (512) and its shank (514) comprises a threading.

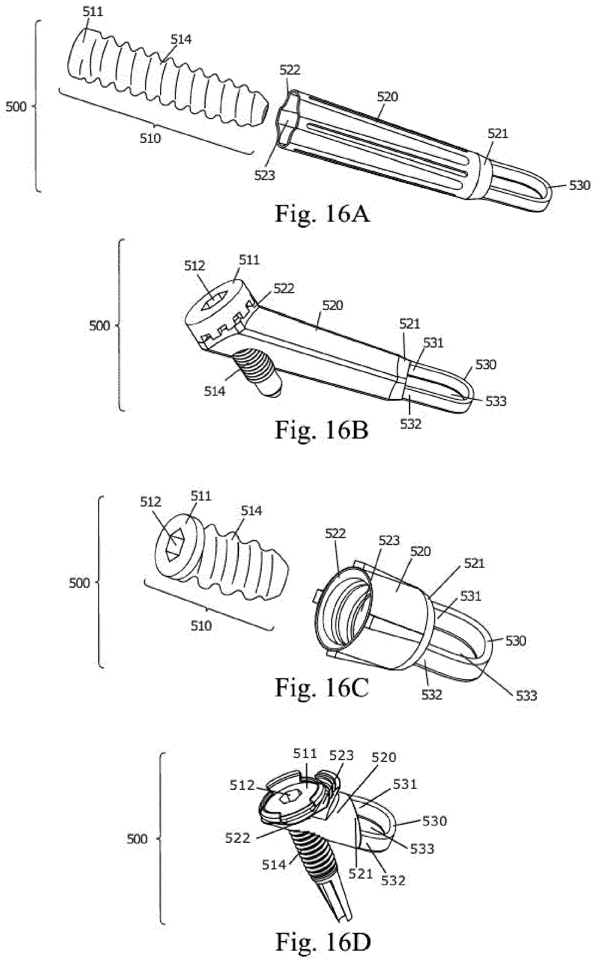

### Second fastening device: first embodiment of the second fastening element (520): Figures 15A to 15C and 16A to 16D

Figures 15A to 15C and 16A to 16D show a first embodiment of the second medical fastening device (500) of the invention. To clarify in the example of Figures 15A to 15C and 16A to 16D, it has been indicated that the second fastening element (520) has a first end (521) and a second end (522); furthermore, the second fastening element (520) comprises a fastening conduit (523) located at its second end (522) intended for housing the first fastening element (510).

As can be seen, the second fastening element (520) comprises a distal appendage (530) configuring a suspension element, with a first end (531) and a second end (532) connected to the first end (521) of the second fastening element (520), such that a non-adjustable hole or closed loop (533) intended for the suspension of a bent end of the graft in the bone tunnel is configured.

Advantageously, the devices of Figures 15A to 15C and 16A to 16D allow reproducing the diagrams of Figure 13A, 3-strand configuration of the graft, and Figure 14A, 4-strand configuration of the graft, by means of methods of use similar to those described in the first invention.

### Second fastening device: second embodiment of the second fastening element (520) configuring an adjustable loop; Figure 15D

Figure 15D shows a second embodiment of the second medical fastening device (500) of the invention. As can be seen, the second embodiment is similar to the first embodiment, with the exception that the distal appendage (530) is configured as an adjustable loop (533).

As can be seen in Figure 15D, in this particular embodiment the distal appendage (530) comprises a strip, cord, or band, with a first end (531) connected to the first end (521) of the second fastening element (520) and a second fastening-free end (532). The second end (532) can be configured such that it goes back to the second fastening element (520) configuring an adjustable loop (533) as shown in Figure 15D.

The length of the adjustable loop is fastened by means of the first fastening element (510), with the adjustable loop (533) being intended for the suspension of a bent end of the graft (10) in the bone tunnel or bone canal. Said fastening is adjustable from a first position without tension, to a final position with ideal tension of the suspended graft.

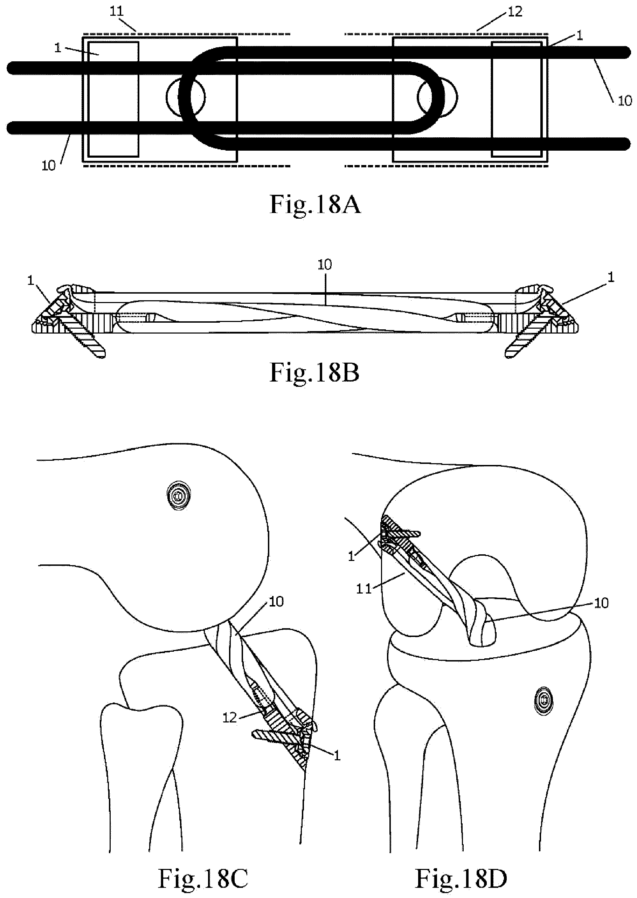

### Surgical method: restauration of a damaged anterior cruciate ligament (ACL) by means of a first and a second fastening devices (1); Figures 18A-18D.

In figures 18A-18D an example of a surgical method for restoring a damaged anterior cruciate ligament (ACL) is shown. With this surgical method, the restauration of the ACL is carried out by means of a first and a second fastening devices (1) with suspension holes (273).

Advantageously, providing a device (1) with a suspension hole (273) for each of the bone tunnels - tibial and femoral bone tunnels - allows suspending a first graft (10) in one of the bone tunnels and suspending a second graft (10) in the opposite bone tunnel to fasten, in both cases, the free ends of each graft (10) with the device (1) placed in the opposite side where the graft (10) is suspended, as shown in figure 18B, 18C and 18D.

The surgical method comprises the following steps:
1) performing a femoral bone tunnel, with intra-articular entrance of the femoral bone tunnel in the insertion site of the original ACL,
2) performing a tibial bone tunnel, with intra-articular entrance of the tibial bone tunnel in the insertion site of the original ACL,
3) suspending, from the suspension hole (273) of the first fastening device (1), the bent end of a first graft (10),
4) introducing the free ends of the first graft (10) through the extra-articular entrance of the femoral bone tunnel until reaching, throughout the intra-articular cavity of the knee joint, the intra-articular entrance of the tibial bone tunnel until the free ends of the first graft (10) protrude from the extra-articular entrance of the tibial bone tunnel,
5) suspending, from the suspension hole (273) of the second fastening device (1), the bent end of a second graft (10),
6) introducing the free ends of the second graft (10) through the extra-articular entrance of the tibial bone tunnel until reaching, throughout the intra-articular cavity of the knee joint, the intra-articular entrance of the femoral bone tunnel until the free ends of the second graft (10) protrude from the extra-articular entrance of the femoral bone tunnel,
being the free ends of the first and second grafts (1) placed in the extra-articular tibial and femoral entrances, so that each of the free ends can be fastened by the device (1) placed in the opposite side where the graft (10) is suspended, as shown in figure 18C and 18D.

This configuration, additionally, allows twisting both grafts so that the intra-articular segment of the grafts is configured with the anatomical twist that is characteristic of the biomechanics of the original ACL, as it is shown in figure 18C and 18D.

## Claims

1. A medical fastening device (1) for the fastening of a graft (10), wherein the medical fastening device (1) is suitable for being inserted in a bone tunnel (11, 12) of a bone, and wherein said medical fastening device (1) comprises
- a first fastening element (100),
- a second fastening element (200), comprising:
∘ a first through conduit (210) with a first end (211) and a second end (212), wherein said first through conduit (210) is extended along a first longitudinal axis (215), said first through conduit (210) is configured for receiving the graft (10) at its first end (211) and for housing the graft (10) inside same and at its second end (212),
∘ a second fastening conduit (220) with a first end (221) and a second end (222), wherein said second fastening conduit (220) is extended along a second longitudinal axis (225),
wherein the first through conduit (210) and the second fastening conduit (220) are arranged inside the second fastening element (200) such that the first longitudinal axis (215) and the second longitudinal axis (225) define an angle (α) with one another other than 0,
wherein the second end (212) of the first through conduit (210) and the second end (222) of the second fastening conduit (220) are joined defining an annular proximal surface (230),
such that when the first fastening element (100) is housed and fastened in the fastening conduit (220), an annular fastening passage (300) is defined between the first fastening element (100) and the annular proximal surface (230).

2. The device according to claim 1, **characterized in that** the annular proximal surface (230) is prolonged along the entire annular perimeter, defining a proximal cavity (235).

3. The device (1) according to any of the preceding claims, wherein the second fastening element (200) comprises a distal appendage (250, 260, 270, 280) with a first end (251, 261, 271, 281) and a second end (252, 262, 272, 282).

4. The device (1) according to claim 3, wherein the second end (252, 262, 272, 282) of the distal appendage (250, 260, 270, 280) is connected to the first end (211) of the first through conduit (210), the distal appendage (250, 260, 270, 280) projecting from the first end (211) of the first through conduit (210).

5. The device (1) according to claim 3 or 4, wherein
- the second end (282) of the distal appendage (280) is additionally housed in the first through conduit (210) dividing said first through conduit (210) into two sections, and
- the first end (281) projects from the first end (211) of the first through conduit (210).

6. The device (1) according to any of claims 4 or 5, wherein the first end (271, 281) of the distal appendage (270, 280) additionally comprises a hole (273, 283), said hole (273, 283) being intended for the suspension of a bent end of the graft (10), such that when the first fastening element (100) is housed and fastened in the second fastening conduit (220) of the second fastening element (200), the medical fastening device (1) allows fastening at least one free end of the graft (10) while simultaneously at the same time the hole (263) allows maintaining the suspension of the at least one bent end of the graft (10).

7. The device (1) according to claim 3, the distal appendage (240) preferably being a strip, cord, or band,
wherein the first end (241) of the distal appendage (240) is connected to the second fastening element (200), and
wherein the second end (242) of the distal appendage (240) is a free end, such that when the fastening element (100) is housed and fastened in the fastening conduit (220), additionally the second end (242) of the distal appendage (240) is housed in the fastening passage (300), configuring an adjustable loop (245), with the adjustable loop (245) being intended for the suspension of a bent end of the graft (10).

8. The device (1) according to claim 3, the distal appendage (250, 260) preferably being a strip, cord, or band,
wherein the first end (251, 261) and the second end (252, 262) of the distal appendage (250, 260) are connected to the second fastening element (200) configuring a hole (253, 263), the hole (253, 263) being intended for the suspension of a bent end of the graft (10), such that when the first fastening element (100) is housed and fastened in the second fastening conduit (220) of the second fastening element (200), the medical fastening device (1) allows fastening at least one free end of the graft (10) while simultaneously at the same time the hole (253, 263) maintains the suspension of the at least one bent end of the graft (10).

9. The device (1) according to any of the preceding claims, wherein the first fastening element is a screw (110) with a screw shaft and a head, and a washer (120), and wherein
- the head of the screw comprises a circular step (111) concentric to the shaft of the screw (110), and
- the washer (120) comprises an inner circular step (121) with a reciprocal shape with respect to the circular step (111) concentric to the shaft of the screw (110), wherein said inner circular step (121) is located in the area of the washer (120) for receiving the head of the screw.

10. The device (1) according to any of the preceding claims, wherein the second fastening element (200) comprises a movable junction area (290) movable between the first through conduit (210) and the second fastening conduit (220), and wherein said movable junction area is configured for:
- moving to a first position inside the second fastening conduit (220), and
- moving to a second position inside the first through conduit (210).

11. The device (1) according to any of the preceding claims 1-10, wherein the angle (α) is comprised between 30° and 60°.

12. The device (1) according to any of the preceding claims, wherein the second end (222) of the second fastening element (200) additionally comprises at least one flange (231) located on an axis perpendicular to the second longitudinal axis (225).

13. The device (1) according to claim 12, wherein the second end (222) of the second fastening element (200) comprises two flanges (231) located on an axis perpendicular to the second longitudinal axis (225).

14. A system (400) for inserting a medical fastening device, comprising
- a medical fastening device (1) according to any of the preceding claims,
- an inserter (450), comprising
o a first end (410) with a reciprocal shape with respect to the shape of the second fastening element (200) and configured for engaging the second end (212) of the second fastening element (200),
o a handle (430) located at a second end (420) of the inserter (450), and
- a coupling screw (440).

## Patentansprüche

1. Medizinische Befestigungsvorrichtung (1) zur Befestigung eines Transplantats (10), wobei die medizinische Befestigungsvorrichtung (1) zum Einfügen in einen Knochentunnel (11, 12) eines Knochens geeignet ist, und wobei die medizinische Befestigungsvorrichtung (1) umfasst:
- ein erstes Befestigungselement (100),
- ein zweites Befestigungselement (200), umfassend:
∘ eine erste Durchleitung (210) mit einem ersten Ende (211) und einem zweiten Ende (212), wobei sich die erste Durchleitung (210) entlang einer ersten Längsachse (215) erstreckt, wobei die erste Durchleitung (210) dazu konfiguriert ist, das Transplantat (10) an ihrem ersten Ende (211) aufzunehmen und das Transplantat (10) innerhalb derselben und an ihrem zweiten Ende (212) unterzubringen,
∘ eine zweite Befestigungsleitung (220) mit einem ersten Ende (221) und einem zweiten Ende (222), wobei sich die zweite Befestigungsleitung (220) entlang einer zweiten Längsachse (225) erstreckt,
wobei die erste Durchleitung (210) und die zweite Befestigungsleitung (220) innerhalb des zweiten Befestigungselements (200) so angeordnet sind, dass die erste Längsachse (215) und die zweite Längsachse (225) einen Winkel (a) zueinander definieren, der ungleich 0 ist,
wobei das zweite Ende (212) der ersten Durchleitung (210) und das zweite Ende (222) der zweiten Befestigungsleitung (220) zusammengefügt sind und eine ringförmige proximale Oberfläche (230) definieren,
so dass, wenn das erste Befestigungselement (100) in der Befestigungsleitung (220) untergebracht und befestigt ist, ein ringförmiger Befestigungsdurchgang (300) zwischen dem ersten Befestigungselement (100) und der ringförmigen proximalen Oberfläche (230) definiert ist.

2. Vorrichtung gemäß Anspruch 1, **dadurch gekennzeichnet, dass** die ringförmige proximale Oberfläche (230) entlang des gesamten ringförmigen Umfangs verlängert ist und eine proximale Kavität (235) definiert.

3. Vorrichtung (1) gemäß einem der vorhergehenden Ansprüche, wobei das zweite Befestigungselement (200) einen distalen Fortsatz (250, 260, 270, 280) mit einem ersten Ende (251, 261, 271, 281) und einem zweiten Ende (252, 262, 272, 282) umfasst.

4. Vorrichtung (1) gemäß Anspruch 3, wobei das zweite Ende (252, 262, 272, 282) des distalen Fortsatzes (250, 260, 270, 280) mit dem ersten Ende (211) der ersten Durchleitung (210) verbunden ist, wobei der distale Fortsatz (250, 260, 270, 280) aus dem ersten Ende (211) der ersten Durchleitung (210) herausragt.

5. Vorrichtung (1) gemäß Anspruch 3 oder 4, wobei
- das zweite Ende (282) des distalen Fortsatzes (280) zusätzlich in der ersten Durchleitung (210) untergebracht ist, wodurch die erste Durchleitung (210) in zwei Abschnitte geteilt wird, und
- das erste Ende (281) aus dem ersten Ende (211) der ersten Durchleitung (210) herausragt.

6. Vorrichtung (1) gemäß einem der Ansprüche 4 oder 5, wobei das erste Ende (271, 281) des distalen Fortsatzes (270, 280) zusätzlich ein Loch (273, 283) umfasst, wobei das Loch (273, 283) für die Aufhängung eines gebogenen Endes des Transplantats (10) vorgesehen ist, so dass, wenn das erste Befestigungselement (100) in der zweiten Befestigungsleitung (220) des zweiten Befestigungselements (200) untergebracht und befestigt ist, die medizinische Befestigungsvorrichtung (1) die Befestigung mindestens eines freien Endes des Transplantats (10) erlaubt, während gleichzeitig das Loch (263) es erlaubt, die Aufhängung des mindestens einen gebogenen Endes des Transplantats (10) aufrecht zu erhalten.

7. Vorrichtung (1) gemäß Anspruch 3, wobei der distale Fortsatz (240) vorzugsweise ein Streifen, eine Schnur oder ein Band ist,
wobei das erste Ende (241) des distalen Fortsatzes (240) mit dem zweiten Befestigungselement (200) verbunden ist, und
wobei das zweite Ende (242) des distalen Fortsatzes (240) ein freies Ende ist, so dass, wenn das Befestigungselement (100) in der Befestigungsleitung (220) untergebracht und befestigt ist, zusätzlich das zweite Ende (242) des distalen Fortsatzes (240) in dem Befestigungsdurchgang (300) untergebracht ist, wodurch eine einstellbare Schlaufe (245) konfiguriert wird, wobei die einstellbare Schlaufe (245) für die Aufhängung eines gebogenen Endes des Transplantats (10) vorgesehen ist.

8. Vorrichtung (1) gemäß Anspruch 3, wobei der distale Fortsatz (250, 260) vorzugsweise ein Streifen, eine Schnur oder ein Band ist,
wobei das erste Ende (251, 261) und das zweite Ende (252, 262) des distalen Fortsatzes (250, 260) mit dem zweiten Befestigungselement (200) verbunden sind und dabei ein Loch (253, 263) bilden, wobei das Loch (253, 263) für die Aufhängung eines gebogenen Endes des Transplantats (10) vorgesehen ist, so dass, wenn das erste Befestigungselement (100) in der zweiten Befestigungsleitung (220) des zweiten Befestigungselements (200) untergebracht und befestigt ist, die medizinische Befestigungsvorrichtung (1) die Befestigung mindestens eines freien Endes des Transplantats (10) erlaubt, während gleichzeitig das Loch (253, 263) die Aufhängung des mindestens einen gebogenen Endes des Transplantats (10) aufrechterhält.

9. Vorrichtung (1) gemäß einem der vorhergehenden Ansprüche, wobei das erste Befestigungselement eine Schraube (110) mit einem Schraubenschaft und einem Kopf und einer Unterlegscheibe (120) ist, und wobei
- der Kopf der Schraube eine kreisförmige Stufe (111) umfasst, die konzentrisch zum Schaft der Schraube (110) ist, und
- die Unterlegscheibe (120) eine innere kreisförmige Stufe (121) umfasst mit einer reziproken Form in Bezug auf die kreisförmige Stufe (111), die konzentrisch zum Schaft der Schraube (110) ist, wobei die innere kreisförmige Stufe (121) in dem Gebiet der Unterlegscheibe (120) zum Aufnehmen des Schraubenkopfes verortet ist.

10. Vorrichtung (1) gemäß einem der vorhergehenden Ansprüche, wobei das zweite Befestigungselement (200) eine bewegliche Verbindungsfläche (290) umfasst, die zwischen der ersten Durchleitung (210) und der zweiten Befestigungsleitung (220) beweglich ist, und wobei die bewegliche Verbindungsfläche dazu konfiguriert ist, um:
- sich in eine erste Position innerhalb der zweiten Befestigungsleitung (220) zu bewegen, und
- sich in eine zweite Position innerhalb der ersten Durchleitung (210) zu bewegen.

11. Vorrichtung (1) gemäß einem der vorhergehenden Ansprüche 1-10, wobei der Winkel (a) zwischen 30° und 60° umfasst.

12. Vorrichtung (1) gemäß einem der vorhergehenden Ansprüche, wobei das zweite Ende (222) des zweiten Befestigungselements (200) zusätzlich mindestens einen Flansch (231) umfasst, der auf einer Achse senkrecht zur zweiten Längsachse (225) verortet ist.

13. Vorrichtung (1) gemäß Anspruch 12, wobei das zweite Ende (222) des zweiten Befestigungselements (200) zwei Flansche (231) umfasst, die auf einer Achse senkrecht zur zweiten Längsachse (225) verortet sind.

14. System (400) zum Einfügen einer medizinischen Befestigungsvorrichtung, umfassend
- eine medizinische Befestigungsvorrichtung (1) gemäß einem der vorhergehenden Ansprüche,
- einen Einfüger (450), umfassend:
∘ ein erstes Ende (410) mit einer reziproken Form in Bezug auf die Form des zweiten Befestigungselements (200) und dazu konfiguriert, mit dem zweiten Ende (212) des zweiten Befestigungselements (200) in Eingriff zu kommen,
∘ einem Griff (430), der an einem zweiten Ende (420) des Einführers (450) verortet ist, und
- eine Kopplungsschraube (440).

## Revendications

1. Un dispositif médical de fixation (1) pour la fixation d'un greffon (10), le dispositif médical de fixation (1) étant adapté pour être inséré dans un tunnel osseux (11, 12) d'un os, et ledit dispositif médical de fixation (1) comprenant
- un premier élément de fixation (100),
- un deuxième élément de fixation (200), comprenant :
o un premier conduit traversant (210) avec une première extrémité (211) et une deuxième extrémité (212), ledit premier conduit traversant (210) s'étendant le long d'un premier axe longitudinal (215), ledit premier conduit traversant (210) étant configuré pour recevoir le greffon (10) à sa première extrémité (211) et pour loger le greffon (10) à l'intérieur de lui-même et à sa deuxième extrémité (212),
o un deuxième conduit de fixation (220) avec une première extrémité (221) et une deuxième extrémité (222), ledit deuxième conduit de fixation (220) s'étendant selon un deuxième axe longitudinal (225),
le premier conduit traversant (210) et le deuxième conduit de fixation (220) étant agencés à l'intérieur du deuxième élément de fixation (200) de sorte que le premier axe longitudinal (215) et le deuxième axe longitudinal (225) définissent l'un avec l'autre un angle (α) autre que de 0,
la deuxième extrémité (212) du premier conduit traversant (210) et la deuxième extrémité (222) du deuxième conduit de fixation (220) étant jointes définissant une surface proximale annulaire (230),
de sorte que, lorsque le premier élément de fixation (100) est logé et fixé dans le conduit de fixation (220), un passage annulaire de fixation (300) est défini entre le premier élément de fixation (100) et la surface proximale annulaire (230).

2. Le dispositif selon la revendication 1, **caractérisé en ce que** la surface proximale annulaire (230) se prolonge sur tout le périmètre annulaire, définissant une cavité proximale (235).

3. Le dispositif (1) selon l'une quelconque des revendications précédentes, dans lequel le deuxième élément de fixation (200) comprend un appendice distal (250, 260, 270, 280) ayant une première extrémité (251, 261, 271, 281) et une deuxième extrémité (252, 262, 272, 282).

4. Le dispositif (1) selon la revendication 3, dans lequel la deuxième extrémité (252, 262, 272, 282) de l'appendice distal (250, 260, 270, 280) est reliée à la première extrémité (211) du premier conduit traversant (210), l'appendice distal (250, 260, 270, 280) dépassant de la première extrémité (211) du premier conduit traversant (210) .

5. Le dispositif (1) selon la revendication 3 ou la revendication 4, dans lequel
- la deuxième extrémité (282) de l'appendice distal (280) est en outre logée dans le premier conduit traversant (210), divisant ledit premier conduit traversant (210) en deux tronçons, et
- la première extrémité (281) dépasse de la première extrémité (211) du premier conduit traversant (210).

6. Le dispositif (1) selon l'une quelconque des revendications 4 ou 5, dans lequel la première extrémité (271, 281) de l'appendice distal (270, 280) comprend en outre un trou (273, 283), ledit trou (273, 283) étant destiné à la suspension d'une extrémité coudée du greffon (10), de sorte que lorsque le premier élément de fixation (100) est logé et fixé dans le deuxième conduit de fixation (220) du deuxième élément de fixation (200), le dispositif médical de fixation (1) permet de fixer au moins une extrémité libre du greffon (10) tandis que, simultanément, le trou (263) permet de maintenir la suspension de ladite au moins une extrémité coudée du greffon (10).

7. Le dispositif (1) selon la revendication 3, l'appendice distal (240) étant de préférence une bande, un cordon ou un ruban,
dans lequel la première extrémité (241) de l'appendice distal (240) est reliée au deuxième élément de fixation (200), et
dans lequel la deuxième extrémité (242) de l'appendice distal (240) est une extrémité libre, de sorte que, lorsque l'élément de fixation (100) est logé et fixé dans le conduit de fixation (220), la deuxième extrémité (242) de l'appendice distal l'appendice (240) est additionnellement logée dans le passage de fixation (300), formant une boucle réglable (245), la boucle réglable (245) étant destinée à la suspension d'une extrémité coudée du greffon (10).

8. Le dispositif (1) selon la revendication 3, l'appendice distal (250, 260) étant de préférence une bande, un cordon ou un ruban,
dans lequel la première extrémité (251, 261) et la deuxième extrémité (252, 262) de l'appendice distal (250, 260) sont reliées au deuxième élément de fixation (200), formant un trou (253, 263), le trou (253, 263) étant destiné à la suspension d'une extrémité coudée du greffon (10), de sorte que lorsque le premier élément de fixation (100) est logé et fixé dans le deuxième conduit de fixation (220) du deuxième élément de fixation (200), le dispositif médical de fixation (1) permet de fixer au moins une extrémité libre du greffon (10) tandis que, simultanément, le trou (253, 263) maintient la suspension de ladite au moins une extrémité coudée du greffon (10).

9. Le dispositif (1) selon l'une quelconque des revendications précédentes, dans lequel le premier élément de fixation est une vis (110) avec un corps de vis et une tête de vis, et une rondelle (120), et dans lequel
- la tête de la vis comporte un épaulement circulaire (111) concentrique au corps de la vis (110), et
- la rondelle (120) comporte un épaulement circulaire intérieur (121) de forme inversée par rapport à l'épaulement circulaire (111) concentrique au corps de la vis (110), ledit épaulement circulaire intérieur (121) étant situé au niveau de la rondelle (120) de façon à recevoir la tête de la vis.

10. Le dispositif (1) selon l'une quelconque des revendications précédentes, dans lequel le deuxième élément de fixation (200) comprend une zone de jonction mobile (290) mobile entre le premier conduit traversant (210) et le deuxième conduit de fixation (220), et dans lequel ladite zone de jonction mobile est configurée pour :
- se déplacer vers une première position à l'intérieur du deuxième conduit de fixation (220), et
- se déplacer vers une deuxième position à l'intérieur du premier conduit traversant (210).

11. Le dispositif (1) selon l'une quelconque des revendications précédentes 1 à 10, dans lequel l'angle (α) est compris entre 30° et 60°.

12. Le dispositif (1) selon l'une quelconque des revendications précédentes, dans lequel la deuxième extrémité (222) du deuxième élément de fixation (200) comprend en outre au moins une collerette (231) située sur un axe perpendiculaire au deuxième axe longitudinal (225).

13. Le dispositif (1) selon la revendication 12, dans lequel la deuxième extrémité (222) du deuxième élément de fixation (200) comprend deux brides (231) situées sur un axe perpendiculaire au deuxième axe longitudinal (225).

14. Un système (400) d'insertion d'un dispositif médical de fixation, comprenant
- un dispositif médical de fixation (1) selon l'une quelconque des revendications précédentes,
- un dispositif d'insertion (450), comprenant
∘ une première extrémité (410) ayant une forme inversée par rapport à la forme du deuxième élément de fixation (200) et configurée pour venir en engagement avec la deuxième extrémité (212) du deuxième élément de fixation (200),
o une poignée (430) située au niveau d'une deuxième extrémité (420) du dispositif d'insertion (450), et
- une vis de liaison (440).
